(19)

**Europäisches Patentamt**
**European Patent Office**
**Office européen des brevets**

(11) **EP 4 186 928 A1**

(12) **EUROPEAN PATENT APPLICATION**
published in accordance with Art. 153(4) EPC

(43) Date of publication:
**31.05.2023 Bulletin 2023/22**

(21) Application number: **21838465.9**

(22) Date of filing: **08.07.2021**

(51) International Patent Classification (IPC):
*C07K 19/00* (2006.01)    *C12N 15/62* (2006.01)
*A61K 38/16* (2006.01)    *A61P 35/00* (2006.01)

(52) Cooperative Patent Classification (CPC):
**A61K 38/16; A61P 35/00; C07K 19/00; C12N 15/62**

(86) International application number:
**PCT/CN2021/105187**

(87) International publication number:
**WO 2022/007885 (13.01.2022 Gazette 2022/02)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.07.2020  CN 202010650886**
**08.07.2020  CN 202010651373**

(71) Applicant: **Nanjing Normal University
Nanjing, Jiangsu 210023 (CN)**

(72) Inventors:
• **SHEN, Jian
  Nanjing, Jiangsu 210023 (CN)**
• **ZHOU, Jiahong
  Nanjing, Jiangsu 210023 (CN)**

(74) Representative: **Nederlandsch Octrooibureau
P.O. Box 29720
2502 LS The Hague (NL)**

Remarks:
The complete document including Reference
Table(s) and the Sequence Listing(s) can be
downloaded from the EPO website

(54) **FUSION POLYPEPTIDE AND POLYPEPTIDE DIMER, AND USE THEREOF**

(57)    The present invention provides a fusion polypeptide, comprising a carrier protein and a polypeptide of interest, wherein the carrier protein has a plurality of coil domains linked by a ring, the polypeptide of interest is inserted in the ring of the carrier protein, and the carrier protein masks a site of interest on the polypeptide of interest, thereby blocking the accessibility of the site. The present invention also provides a polypeptide dimer, comprising a first polypeptide and a second polypeptide, wherein the first polypeptide comprises a first dimerization domain and a polypeptide of interest, the second polypeptide comprises a second dimerization domain and a binding domain, and the binding domain can bind to a site of interest on the polypeptide of interest. The present invention also provides a polynucleotide and a vector encoding the fusion polypeptide and polypeptide dimer, and a host cell comprising the polynucleotide and/or vector. Furthermore, the present invention also provides use of the fusion polypeptide and polypeptide dimer of the present invention in the treatment of cancer and/or the activation of immune cells.

EP 4 186 928 A1

**Description**

**Technical Field**

Provided are a fusion polypeptide and a polypeptide dimer. Particularly, provided is a modified immunoregulatory molecule and use thereof for treating a cancer.

**Background**

Regulation of the immune system by a cytokine, such as activation of an immune cell, is a strategy for cancer immunotherapy. However, certain sites on the cytokines influnece their functions (e.g., immunomodulatory effect).

For example, interleukin-2 (IL-2) can regulate the function of immune cells. After binding to receptors IL2Rα (CD25), IL2Rβ (CD 122) and IL2Rγ (CD 132), IL-2 can activate downstream signaling pathways, including JAK1 and JAK3 kinases and transcription factor STATS, to stimulate activation and proliferation of immune cells (such as T cells and natural killer (NK) cells). IL-2 can perform signal transduction through CD25/CD122/CD132 trimer or activate signaling pathway through CD122/CD132 dimer. Binding of CD25 to IL-2 will change the conformation of IL-2, increasing its affinity for CD122/CD132 dimer by 100-fold (KD value increased from 1nM to 10pM). CD122/CD132 dimer is mainly expressed on the surface of CD8+ memory T cells and NK cells, while CD25/CD122/CD132 trimer is abundantly expressed on the surface of regulatory T cells (Treg) that play an immunosuppressive role. Therefore, the role of IL-2 is to stimulate or inhibit the activation of the immune system, depending on the activation of different immune cell types.

IL-2 is also a class of potential drugs for cancer immunotherapy that has attracted much attention. Recombinant IL-2 (Aldesleukin) was approved by the U.S. Food and Drug Administration (FDA) in 1998 for the treatment of metastatic melanoma and renal cancer and is the only IL-2-based drug approved so far. However, only 10% of patients respond, and its side effects are significant, mainly due to the two-sided nature of IL-2, requiring a higher dose to stimulate immune activation, but the higher dose can cause side effects like capillary leakage syndrome.

Interleukin-15 (IL-15) can also activate the immune response and play an important role in the differentiation and proliferation of T cells and NK cells, as well as the development of dendritic cells. IL-15 shares a similar mechanism with IL-2 and activates downstream signaling pathways (JAK1/JAK3 and STAT3/STATS) by binding to CD122/CD132 receptor dimer, but its α receptor is different from IL-2, being a unique IL-15Rα (CD215) receptor. It is generally believed that after binding to the IL-15R α receptor on the cell membrane, IL-15 is presented to the CD122/CD132 receptor with high affinity as "trans" (cell-cell contact) or "cis" (cis, on the same cell). It has also been found that IL-15 can also bind to CD122/CD132 with moderate affinity and function without binding to IL-15Rα.

It is recently reported (Alexandra Berger *et al.,* 2019 J for ImmunoTherapy for Cancer) that IL-15 alone was more effective than the IL-15/IL-15Rα receptor extracellular domain complex in a mouse tumor model for durable anti-tumor activity. The applicant believes that this may be because although the complex can stimulate immunity quickly and potently, it will also cause immune cells exhaustion more quickly and reduce response durability. Those skilled in the art also know that strong immune stimulation may bring about safety issues.

In addition, cytokines, such as IL-2 and IL-15, have short *in vivo* half-lives and require continuous injection.

Therefore, it is desirable to develop an engineered recombinant cytokine such as IL-2 and IL-15 that can specifically activate immunity and have an extended half-life, have improved activity, have a more durable ability to activate immunity, and/or have enhanced safety, thereby improving the clinical application and commercial conversion value.

**Summary**

In the first aspect, provided is a fusion polypeptide, comprising a carrier protein and a polypeptide of interest, wherein the carrier protein has a plurality of helical domains linked with loops, the polypeptide of interest is inserted into a loop of the carrier protein, the carrier protein shields a site of interest on the polypeptide of interest, thereby blocking the accessibility of the site.

In some embodiments, the polypeptide of interest is derived from a cytokine of the four α-helical bundle cytokine family, the cytokine comprises four α-helix bundles of, from N-terminal to C-terminal, helical bundle 1 (H1), helical bundle 2 (H2), helical bundle 3 (H3) and helical bundle 4 (H4).

In some embodiments, the polypeptide of interest is a cytokine of the circularly permutated four α-helical bundle cytokine family, comprising four α-helical bundles of, from N-terminal to C-terminal, H2, H3, H4 and H1; H3, H4, H1 and H2; or H4, H1, H2 and H3.

In some embodiments, the amino acid in the circularly permutated cytokine corresponding to N-terminal of the uncircularly permutated cytokine is linked to the amino acid corresponding to C-terminal of the uncircularly permutated cytokine via a linker. In some embodiments, the linker is a GS linker or a polyglycine linker having a length of 1-10 amino acids.

In some embodiments, the polypeptide of interest is selected from the group consisting of a circularly permutated IL-2 and a circularly permutated IL-15.

In some embodiments, the circularly permutated IL-2 comprises four α-helical bundles of, from N-terminal to C-terminal, H3, H4, H1 and H2, or H4, H1, H2 and H3. In some embodiments, the circularly permutated IL-2 comprises an amino acid sequence of SEQ ID NO: 2, 3, 4 or 5. In some embodiments, the site of interest is a CD25 binding site.

In some embodiments, the circularly permutated IL-15 comprises four α-helical bundles of, from N-terminal to C-terminal, H3, H4, H1 and H2. In some embodiments, the circularly permutated IL-15 comprises an amino acid sequence of SEQ ID NO: 7. In some embodiments, the site of interest is a CD215 binding site.

In some embodiments, the carrier protein is an albumin, preferably a human serum albumin (HSA). In some embodiments, the loop is selected from the group consisting of loops at D56-L66, A92-P96, D129-E131, Q170-A172, K281-L283, V293-L305, E311-S312, E321-A322, A362-D365, L398-E400, K439-R445, E465-D471, P537-E542 and A561-T566, the positions are numbered by reference to SEQ ID NO: 16. In some embodiments, the polypeptide of interest is a circularly permutated IL-2, the loop is selected from the group consisting of loops at D56-L66, V293-L305 and A362-D365 of the HSA. In some embodiments, the insertion site of the polypeptide of interest is selected from the group consisting of D56, A300, C361 and A362 of the HSA.

In some embodiments, provided is a fusion polypeptide, comprising a carrier protein HSA and a circularly permutated IL-2, wherein the circularly permutated IL-2 comprises four α-helical bundles of, from N-terminal to C-terminal, H3, H4, H1 and H2, or H4, H1, H2 and H3; and wherein the circularly permutated IL-2 is inserted into a loop of the HSA, the loop is selected from the group consisting of loops at D56-L66, A92-P96, D129-E131, Q170-A172, K281-L283, V293-L305, E311-S312, E321-A322, A362-D365, L398-E400, K439-R445, E465-D471, P537-E542, A561-T566, the positions are numbered by reference to SEQ ID NO: 16, the HSA shields the CD25 binding site of the circularly permutated IL-2, thereby blocking the accessibility of the site. In some embodiments, the circularly permutated IL-2 comprises an amino acid sequence of SEQ ID NO: 2, 3, 4 or 5. In some embodiments, the loop is selected from the group consisting of loops at D56-L66, V293-L305 and A362-D365 of the HSA. In some embodiments, the insertion site of the circularly permutated IL-2 is selected from the group consisting of D56, A300, C361 and A362 of the HSA.

In some embodiments, provided is a fusion polypeptide, comprising a carrier protein HSA and a circularly permutated IL-15, wherein the circularly permutated IL-15 comprises four α-helical bundles of, from N-terminal to C-terminal, H3, H4, H1 and H2; and wherein the circularly permutated IL-15 is inserted into a loop of the HSA, the loop is selected from the group consisting of loops at D56-L66, A92-P96, D129-E131, Q170-A172, K281-L283, V293-L305, E311-SS312, E321-A322, A362-D365, L398-E400, K439-R445, E465-D471, P537-E542 and A561-T566, the positions are renumbered by reference to SEQ ID NO: 16, the HSA shields the CD215 binding site of the circularly permutated IL-15, thereby blocking the accessibility of the site. In some embodiments, the circularly permutated IL-15 comprises an amino acid sequence of SEQ ID NO: 7.

In some embodiments, provided is a fusion polypeptide, comprising an amino acid sequence of one of SEQ ID NOs: 8-11.

Provided is also a pharmaceutical composition, comprising the fusion polypeptide according to the present disclosure.

In the second aspect, provided is a polypeptide dimer, comprising a first polypeptide and a second polypeptide,

wherein the first polypeptide comprises a first dimerization domain and a polypeptide of interest, wherein the polypeptide of interest is located at the first end of the first dimerization domain,
wherein the second polypeptide comprises a second dimerization domain and a binding domain, wherein the binding domain is located at the first end of the second dimerization domain,
wherein the first polypeptide forms a dimer with the second polypeptide through the first dimerization domain and the second dimerization domain, and wherein the first end of the first dimerization domain is adjacent to the first end of the second dimerization domain, the binding domain is capable of binding to the site of interest on the polypeptide of interest.

In some embodiments, the polypeptide of interest is derived from a cytokine of the four α-helical bundle cytokine family, the cytokine comprises four α-helix bundles of, from N-terminal to C-terminal, helical bundle 1 (H1), helical bundle 2 (H2), helical bundle 3 (H3) and helical bundle 4 (H4). In some embodiments, the polypeptide of interest is a cytokine of the circularly permutated four α-helical bundle cytokine family, comprising four α-helical bundles of, from N-terminal to C-terminal, H2, H3, H4 and H1; H3, H4, H1 and H2; or H4, H1, H2 and H3.

In some embodiments, the amino acid in the circularly permutated cytokine corresponding to N-terminal of the natural cytokine is linked to the amino acid corresponding to C-terminal of the natural cytokine via a linker. In some embodiments, the linker is a GS linker or a polyglycine linker having a length of 1-10 amino acids.

In some embodiments, the polypeptide of interest is a circularly permutated IL-2 or a circularly permutated IL-15.

In some embodiments, the circularly permutated IL-2 comprises four α-helical bundles of, from N-terminal to C-terminal, H3, H4, H1 and H2, or H4, H1, H2 and H3. In some embodiments, the circularly permutated IL-2 comprises an amino acid sequence of SEQ ID NO: 21. In some embodiments, the site of interest is a CD25 binding site, the binding domain is an extracellular domain of CD25.

In some embodiments, the circularly permutated IL-15 comprises four α-helical bundles of, from N-terminal to C-terminal, H3, H4, H1 and H2. In some embodiments, the circularly permutated IL-15 comprises an amino acid sequence of SEQ ID NO: 22. In some embodiments, the site of interest is a CD215 binding site, the binding domain is an extracellular domain of CD215.

In some embodiments, the first and second dimerization domains comprise heavy chain constant regions CH2 and CH3 of immunoglobulin (Ig). In some embodiments, the Ig is human Ig, for example human IgG1. In some embodiments, the first dimerization domain forms Fc region of the human IgG1 with the second dimerization domain. In some embodiments, the Fc region of the human IgG1 is modified. In some embodiments, the first dimerization domain comprises an amino acid sequence of SEQ ID NO: 23, and the second dimerization domain comprises an amino acid sequence of SEQ ID NO: 24; or the first dimerization domain comprises an amino acid sequence of SEQ ID NO: 24, and the second dimerization domain comprises an amino acid sequence of SEQ ID NO: 23.

In some embodiments, the first end of the first dimerization domain is C-terminal, and the first end of the second dimerization domain is C-terminal.

In some embodiments, the polypeptide dimer according to the present disclosure comprises a first polypeptide and a second polypeptide,

wherein the first polypeptide comprises a first chain of the Fc region of the human IgG1 and a circularly permutated IL-2, the circularly permutated IL-2 is linked to C-terminal of the first chain of the Fc region of the human IgG1,
wherein the second polypeptide comprises a second chain of the Fc region of the human IgG1 and a CD25 extracellular domain, the CD25 extracellular domain is linked to C-terminal of the second chain of the Fc region of the human IgG1, and
the circularly permutated IL-2 comprises four α-helical bundles of, from N-terminal to C-terminal, H3, H4, H1 and H2; or H4, H1, H2 and H3.

In some embodiments, the circularly permutated IL-2 comprises an amino acid sequence of SEQ ID NO: 21.

In some embodiments, the polypeptide dimer according to the present disclosure comprises a first polypeptide and a second polypeptide,

wherein the first polypeptide comprises a first chain of the Fc region of the human IgG1 and a circularly permutated IL-15, the circularly permutated IL-15 is linked to C-terminal of the first chain of the Fc region of the human IgG1,
wherein the second polypeptide comprises a second chain of the Fc region of the human IgG1 and a CD215 extracellular domain, the CD215 extracellular domain is linked to C-terminal of the second chain of the Fc region of the human IgG1, and
the circularly permutated IL-15 comprises four α-helical bundles of, from N-terminal to C-terminal, H3, H4, H1 and H2.

In some embodiments, the circularly permutated IL-2 comprises an amino acid sequence of SEQ ID NO: 22.

In some embodiments, the first chain comprises an amino acid sequence of SEQ ID NO: 23 and the second chain comprises an amino acid sequence of SEQ ID NO: 24; or the first chain comprises an amino acid sequence of SEQ ID NO: 24 and the second chain comprises an amino acid sequence of SEQ ID NO: 23.

Provided is also a pharmaceutical composition, comprising the polypeptide dimer according to the present disclosure.

In the third aspect, provided is a method for treating a cancer or for activating an immune cell or improving proliferation of an immune cell (e.g., T cell or NK cell), comprising administering a subject in need thereof an effective amount of the fusion polypeptide, polypeptide dimer or pharmaceutical composition according to the present disclosure.

Provided is also use of the fusion polypeptide, polypeptide dimer or pharmaceutical composition according to the present disclosure for the manufacture of a medicament for treating a cancer or for activating an immune cell or improving proliferation of an immune cell (e.g., T cell or NK cell).

In some embodiments, provided is the fusion polypeptide, polypeptide dimer or pharmaceutical composition according to the present disclosure for use in treating a cancer or in activating an immune cell or improving proliferation of an immune cell (e.g., T cell or NK cell).

In the third aspect, provided are a polynucleotide, encoding the fusion polypeptide or polypeptide dimer according to the present disclosure, a vector comprising the polynucleotide, and a host cell comprising the polynucleotide or the vector.

**Brief Description of the Drawings**

Fig. 1 shows the schematic diagram of the three-dimensional structure of wild-type IL-2 binding to its receptor (based on PDB No. 2ERJ), where "loop 1" corresponds to S95-L100 of native IL-2, and "loop 2" corresponds to N50-P54 of native IL-2. Unless otherwise stated, the positions referring to IL-2 herein are numbered by reference to the IL-2 precursor sequence of SEQ ID NO: 1 (UniProt P60568), where amino acid residues 1-21 are the signal peptide sequence, and the sequence of natural IL-2 is amino acid residues 22-153 of SEQ ID NO: 1.

Fig. 2 shows the schematic diagram of the three-dimensional structure of wild-type IL-15 binding to its receptor (based on PDB No. 4GS7), where the "opened loop" corresponds to S102-A105 of natural IL-15. Unless otherwise stated, the positions of IL-15 are numbered by reference to the IL-15 precursor sequence of SEQ ID NO: 6 (UniProt P40933), wherein residues 1-48 are the signal peptide, and the sequence of natural IL-15 is amino acid residues 49-162 of SEQ ID NO: 6.

Fig. 3 shows the schematic diagram of the three-dimensional structure of HSA.

Fig. 4-7 shows the expression of fusion polypeptide according to the present disclosure and the structural model of HSA shielding the CD25 binding site on IL-2.

Fig. 8 and Fig. 9 show the structural model of the polypeptide dimer according to the present disclosure and its expression, and the amino acid sequence of the circularly permutated IL-2 is as shown in SEQ ID NO: 21.

Fig. 10 - Fig.13 show the *in vitro* activity assay of the fusion polypeptide according to the present disclosure.

Fig. 14 and Fig. 15 show the *in vitro* activity assay results of the polypeptide dimer according to the present disclosure, and the amino acid sequence of the circularly permutated IL-2 is as shown in SEQ ID NO: 21.

Fig. 16 shows the experimental results of injecting the fusion polypeptide or polypeptide dimer according to the present disclosure into tumor-bearing mice; A: tumor, 27 days after the first injection; B: curve for the tumor volume over time; and C: curve for mice body weight over time.

Fig. 17 shows the results of immunohistochemical staining of tumors from different groups of mice; A: mean optical density; and B: representative imaging results.

**Detailed Description**

While the present disclosure will be described in view of the embodiments below, it should be understood that they are not intended to limit the present disclosure to those embodiments. Rather, the present disclosure is intended to cover all alternatives, modifications and equivalents as defined in the claims. Those skilled in the art will understand that many methods and materials similar or equivalent to those described herein could be used in the practice of the present disclosure. The present disclosure is not limited to the methods and materials as described. In the event that one or more of the cited literature, patents and similar materials differs from or contradicts with those herein, including but not limited to the defined term, term usage, described techniques or the like, this present disclosure shall prevail. Unless otherwise defined, all technical and scientific terms used herein have the same meaning as commonly understood by those skilled in the art to which present disclosure belongs. Although methods and materials similar or equivalent to those described herein can be used in the practice or testing of the present disclosure, suitable methods and materials are described below. All publications, patent applications, patents and other references mentioned herein are incorporated by reference in their entirety.

I. Definition

As used herein, the term "peptide" refers to a chain of at least two amino acids linked by peptide bonds. The term "polypeptide" is used interchangeably herein with the term "protein" and refers to a chain containing ten or more amino acids residues. All peptide and polypeptide formulae or sequences herein are written from left to right, indicating the direction from the amino terminal to the carboxy terminal.

As used herein, the term "dihedral angle in peptide chain" refers to the angle at which two adjacent peptide bond planes can rotate around the $\alpha$ carbon atom between these two peptide bond planes.

In the context of peptides, the terms "amino acid", "residue" and "amino acid residue" are used interchangeably and include naturally occurring amino acids and non-natural amino acids in proteins. The one-letter and three-letter nomenclature of the naturally occurring amino acids in proteins use the terminology commonly used in the art, and reference can be made to Sambrook, et al. (Molecular Cloning: A Laboratory Manual, 2nd, ed. Cold Spring Harbor Laboratory, Cold Spring Harbor Laboratory Press, Cold Spring Harbor, N.Y., 1989).

| Amino acid | One-letter | Three-letter |
|---|---|---|
| alanine | A | Ala |
| arginine | R | Arg |
| asparagine | N | Asn |
| aspartate | D | Asp |
| cysteine | C | Cys |
| glutamine | Q | Gln |
| glutamate | E | Glu |
| glycine | G | Gly |
| histidine | H | His |
| isoleucine | I | Ile |
| leucine | L | Leu |
| lysine | K | Lys |
| methionine | M | Met |
| phenylalanine | F | Phe |
| proline | P | Pro |
| serine | S | Ser |
| threonine | T | Thr |
| tryptophan | W | Trp |
| tyrosine | Y | Tyr |
| valine | V | Val |

As used herein, the term "polynucleotide" or "nucleic acid molecule" includes a DNA molecule (e.g., cDNA or genomic DNA) and an RNA molecule (e.g., mRNA) and an analog of DNA or RNA produced with a nucleotide analog. The nucleic acid molecule may be single-stranded or double-stranded, preferably double-stranded DNA.

The nucleic acid can be synthesized using nucleotide analogs or derivatives thereof (e.g., inosine or phosphorothioate nucleotides). Such nucleotides can be used, for example, to prepare a nucleic acid with altered base-pairing ability or increased nuclease-resistance.

As used herein, the term "encode" refers to the amino acid sequence of a polynucleotide that directly specifies its protein product. The boundaries of the coding sequence are generally defined by an open reading frame, which usually begins with an ATG initiation codon or another initiation codon like GTG and TTG, and ends with a stop codon like TAA, TAG and TGA. A coding sequence can be DNA, cDNA or a recombinant nucleotide sequence.

As used herein, the term "hybridize" is nucleotide sequences that are at least about 90%, preferably at least about 95%, more preferably at least about 96%, more preferably at least 98% homologous to each other generally remain hybridized to each other under given stringent hybridization and washing conditions.

Those skilled in the art are aware of various conditions for hybridization, such as stringent hybridization conditions and highly stringent hybridization conditions. See, for example, Sambrook et al., 1989, Molecular Cloning, A Laboratory Manual, Cold Spring Harbor Press, N.Y.; and Ausubel et al. (eds.), 1995, Current Protocols in Molecular Biology, John Wiley & Sons, N.Y.

As used herein, "amino acid percent identity" or "amino acid sequence percent identity" refers to comparison of the amino acids of two polypeptides that, when optimally aligned, have approximately the same amino acid percentage as specified. For example, "95% amino acid identity" refers to comparison of the amino acids of two polypeptides that, when optimally aligned, are 95% identical in amino acids.

For the present invention, to determine the identity percentage of two amino acid sequences or two nucleic acid sequences, the sequences are aligned for optimal comparison (e.g., gaps can be introduced in the first amino acid or nucleic acid sequence to be compared with the second amino acid sequence or nucleic acid sequence for optimal alignment). The amino acid residues or nucleotides at corresponding amino acid positions or nucleotide positions are then compared. When a position in the first sequence is occupied by the same amino acid residue or nucleotide at the corresponding position in the second sequence, these molecules are identical at this position. The identity percentage between two sequences is a function of the number of identical positions shared by the sequences (i.e., identity percentage = number of identical positions/total number of positions (i.e., overlapping positions) $\times$ 100). Preferably, the two sequences are of the same length. Those skilled in the art know that different computer programs can be used to determine the identity between two sequences.

As used herein, the term "conservative substitution", also referred to as substitution by a "homologous" amino acid residue, refers to a substitution in which the amino acid residue is replaced by an amino acid residue with a similar side chain, e.g., an amino acid with basic side chain (such as, lysine , arginine and histidine ), an amino acid with acidic side chains (such as, aspartate, glutamate), an uncharged amino acid with polar side chain (such as, glycine, asparagine, glutamine, serine, threonine, tyrosine, cysteine), an amino acid with non-polar side chain (such as, alanine, valine, leucine, isoleucine, proline, phenylalanine, methionine, tryptophan), a β-branched side chain amino acid (such as, threonine, valine, isoleucine) and an amino acid with aromatic side chain (such as, tyrosine, phenylalanine, tryptophan, histidine).

A conservative amino acid substitution generally has minimal effect on the activity of the resulting protein. Such a substitution is described below. A conservative substitution is the replacement of an amino acid with another amino acid which is similar in size, hydrophobicity, charge, polarity, steric characteristic, aromaticity, etc. A substitution is usually conservative when it is desired to fine-tune the properties of a protein.

As used herein, "homologous" amino acid residues refer to amino acid residues that have similar chemical properties with respect to hydrophobicity, charge, polarity, steric characteristics, aromatic characteristics, etc. Examples of amino acids that are homologous to each other include positively-charged amino acids lysine, arginine, histidine; negatively charged amino acids glutamate, aspartate; hydrophobic amino acids glycine, alanine, valine, leucine, isoleucine, proline, phenylalanine; polar amino acids serine, threonine, cysteine, methionine, tryptophan, tyrosine, asparagine, glutamine; aromatic phenylalanine, tyrosine, tryptophan; amino acids with chemically-similar side groups serine and threonine; or glutamine and asparagine; or leucine and isoleucine.

Examples of conservative substitutions of amino acids in proteins include: Ser substituting Ala, Lys substituting Arg, Gln or His substituting Asn, Glu substituting Asp, Ser substituting Cys, Asn substituting Gln, Asp substituting Glu, Pro substituting Gly, Asn or Gln substituting His, Leu or Val substituting Ile, Ile or Val substituting Leu, Arg or Gln substituting Lys, Leu or Ile substituting Met, Met, Leu or Tyr substituting Phe, Thr substituting Ser, Ser substituting Thr, Tyr substituting Trp, Trp or Phe substituting Tyr, and Ile or Leu substituting Val.

As used herein, the term "expression" includes any step involved in the production of a polypeptide, including but not limited to transcription, post-transcriptional modification, translation, post-translational modification and secretion.

As used herein, the term "cytokine" refers to a series of small molecule proteins with a wide range of biological activities that are synthesized and secreted upon stimulation by immune cells (such as monocytes, macrophages, T cells, B cells, NK cells, etc.) and some non-immune cells (endothelial cells, epidermal cells, fibroblasts, etc.). Cytokines generally regulate cell growth and differentiation as well as immune responses, by binding to corresponding receptors.

As used herein, the term "four α-helical bundle cytokine family" refers to cytokines comprising four α-helical bundles in the tertiary structure. A cytokine in the native (wild-type) four α-helical bundle cytokine family comprising four α-helix bundles of, from N-terminal to C-terminal, helical bundle 1 (H1), helical bundle 2 (H2), helical bundle 3 (H3) and helical bundle 4 (H4). The cytokines in the four α-helical bundle cytokine family comprise but not limited to IL-2, IL-4, IL-6, IL-7, IL-9, IL-15, IL-21, G-CSF and GM-CSF.

As used herein, the term "circular permutation" refers to modification of the arrangement order of the four α-helical bundles in the primary structure of the cytokines of the four α-helical bundle cytokine family. For example, a circularly permutated cytokine comprises four α-helix bundles of, from N-terminal to C-terminal, H2, H3, H4 and H1; H3, H4, H1 and H2; or H4, H1, H2 and H3. Circular permutation involves the following design of polypeptides: N- terminal and C- terminal of a polypeptide (original polypeptide, such as wild-type four α-helical bundle cytokine) are fused (either directly or via a linker) to form a circular molecule, and the circular molecule is opened (cleaved or broken) between H1 and H2, H2 and H3 or H3 and H4 to form a new linear polypeptide with N-terminal and C-terminal different from the original polypeptide. The circular permutation maintains the sequence, structure and function of the polypeptide (except the optional linker), while creating new C-terminal and N-terminal at different positions. The circular permutation also includes any process that produces the circularly permutated linear molecules described herein. Typically, the circularly permutated polypeptides are expressed directly as linear molecules without real steps of cyclization and opening.

For a polypeptide, "linker" or "linker sequence" refers to an amino acid sequence covalently linked to the N-terminal and/or C-terminal of the polypeptide. The linker can be used to link N-terminal and C-terminal of the same polypeptide (as in circular permutation), or in the same meaning to link the N-terminal and C-terminal of different polypeptides to form a fusion polypeptide. The linker also involves the polynucleotide encoding the amino acid sequence of the linker. In general, the linker does not have a specific biological activity. However, the amino acids constituting the linker can be selected based on certain properties of the linker or the resulting molecule, such as flexibility, hydrophilicity, net charge or whether it is proteolytically sensitive, and non-immunogenicity.

As used herein, a "wild-type" polypeptide refers to a naturally occurring polypeptide.

As used herein, the term "modification" refers to the modification of a polynucleotide or polypeptide sequence, including but not limited to substitution, deletion, insertion and/or addition of one or more nucleotides or amino acids. Modifications also include chemical modifications that do not alter the sequence of the polynucleotide or polypeptide, such as polynucleotide methylation, polypeptide glycosylation, etc. As used herein, modifications also include circular permutation as described above.

As used herein, the term "opening site" refers to the position in a circular molecule where peptide bonds are eliminated to form new amino-terminal and carboxyl-terminal during circularly permutation process, or the corresponding position in the polynucleotide encoding the polypeptide. The opening site is designated by the positions of a pair of amino acids located between the amino-terminal and carboxyl-terminal of the wild-type polypeptide, and these amino acids become the new amino-terminal and carboxyl-terminal of the circularly permutated polypeptide. For example, in IL-2 (97/96), the new N-terminal corresponds to the residue at position 97 of natural IL-2, and the new C-terminal corresponds to the residue at position 96 of natural IL-2; in IL-15 (105/102), the new N-terminal corresponds to the residue at position 105 of natural IL-15, and the new C-terminal corresponds to the residue at position 102, and residues at positions 103 and 104 of natural IL-15 are removed.

As used herein, the term "receptor" refers to a protein present on the cell surface which binds to a ligand, and also covers a soluble receptor which is not present on the cell surface and has a corresponding cell surface receptor or is associated with a corresponding cell surface receptor. A cell surface receptor is generally composed of different domains or subunits with different functions, such as an extracellular domain containing a region interacting with a ligand, a transmembrane domain anchoring the receptor in the cell membrane, and an intracellular effector domain generating a cellular signal in response to ligand binding (signal transduction). A soluble receptor is typically composed of one or more extracellular domains proteolytically cleaved from the membrane anchoring region.

As used herein, the term "variant" refers to a polypeptide which differs from a reference polypeptide but retains essential properties. A typical variant of a polypeptide has a primary amino acid sequence which differs from the reference polypeptide. The difference is always limited such that the sequences of the reference polypeptide and the variant are overall very similar and identical in many regions. The amino acid sequence of the variant and that of the reference polypeptide may differ by one or more modifications (e.g., substitution, addition and/or deletion). A substituted or inserted amino acid residue may or may not be one encoded by the genetic code. A variant of a polypeptide may be naturally occurring, such as an allelic variant, or be artificially produced. In addition, the term "variant" as used herein encompasses a circularly permutated variant of a polypeptide.

As used herein, the term "carrier protein" refers to a protein which is fused to a molecule of interest (e.g., a polypeptide or hapten) to facilitate the delivery of the protein of interest, prolong its half-life, render the hapten immunogenic. The carrier protein *per se* does not have the biological activity of the molecule of interest. The carrier protein used herein has a plurality of helical structures connected by loop(s), that is, has a "helix-loop-helix" structure, for example, albumin, such as human serum albumin (HSA), albumin binding protein, or the like.

As used herein, the "insertion site" of a polypeptide of interest in a carrier protein refers to the position of residue of the carrier protein closest to N-terminal of the polypeptide of interest in the primary structure after the polypeptide of interest is inserted into the carrier protein.

As used herein, a carrier protein "shielding" a site of interest means that the carrier protein sterically hinders the binding of the site of interest to its receptor, i.e., the carrier protein sterically clashes with the receptor.

As used herein, "accessibility of a site" refers to the ability of a site to contact and bind to its binding partner. When the accessibility of a site is blocked, it cannot contact and bind to its binding partner.

As used herein, the term "immunoglobulin (Ig)" refers to a globulin with antibody activity or chemical structure which is similar to antibody molecule. A natural immunoglobulin is a tetrapeptide chain structure composed of two identical light chains and two identical heavy chains linked via interchain disulfides.

As used herein, the term "Fc fragment" or "Fc region" is part of an immunoglobulin, where Ig is hydrolyzed by papain, cleaving the Ig into two identical Fab fragments and an Fc fragment. The Fc fragment of a native antibody comprises two identical polypeptides linked by disulfides. Each polypeptide comprises two heavy chain constant regions (CH2 and CH3, such as Fc region of IgG), or three heavy chain constant regions (CH2, CH3 and CH4, such as Fc region of IgM and IgE).

As used herein, the term "signal peptide" refers to a short peptide which directs the transfer of a newly synthesized protein to the secretory pathway. Generally, the signal peptide is located at N-terminal of the newly synthesized protein, with a length of, for example, 5-30 amino acid residues. The signal peptide can be removed during protein processing, such that the mature protein does not contain the signal peptide.

As used herein, the term "treatment" refers to a method of obtaining a beneficial or desired result, including but not limited to eradicating or improving the underlying disease being treated. Similarly, the treatment benefit can be obtained by eliminating or improving one or more physiological symptoms related to a basic disease. In this way, although the subject may still suffer from the basic disease, improvements have been observed in the subject.

As used herein, the terms "therapeutically effective amount" and "therapeutically effective dose" refer to the amount of active component. When administered in a single dose or repeated dose, the effective amount can achieve a detectable beneficial effect, including but not limited to the effect on any symptom, aspect, measured parameter or feature of a disease or disorder.

As used herein, the term "dose" refers to the amount administered to a subject once (unit dose) or twice or more within a defined time interval. For example, a dose may refer to the amount administered in one day, two days, one week, two weeks, three weeks or one or more months (for example, by one administration, or two or more administrations).

As used herein, the term "half-life" refers to the time consumed by the reduction of the *in vivo* concentration of a target molecule by 50%. If the target molecule remains *in vivo* in the biological matrix (blood, serum, plasma, tissue) for a longer time than an appropriate control, its half-life will be increased. Compared with the appropriate control, the half-life can be increased by 10%, 20%, 30%, 40%, 50% or more.

Those skilled in the art are familiar with the method of pharmacokinetic analysis and determination of ligand half-life. Detailed information can be found in Kenneth, A et al., Chemical Stability of Pharmaceuticals: A Handbook for Pharmacists and Peters et al., Pharmacokinetc analysis: A Practical Approach (1996). Reference can also be made to "Pharmacokinetics", M Gibaldi & D Perron, published by Marcel Dekker, 2.sup.nd Rev. ex edition (1982).

II. Circular permutation of the polypeptide

Circular permutation refers to change of the order in the primary structure of the four α-helical bundles in the cytokine of the four α-helical bundle cytokine family. For example, the circularly permutated four α-helical bundle cytokine comprises four α-helical bundles of, from N-terminal to C-terminal, H2, H3, H4 and H1; H3, H4, H1 and H2; or H4, H1, H2 and H3. Circular permutation involves the following design of polypeptides: N-terminal and C-terminal of the polypeptide (original polypeptide, such as wild-type four α-helical bundle cytokine) are fused (either directly or via a linker) to form a circular molecule, and the circular molecule is opened between H1 and H2, H2 and H3 or H3 and H4 (cleave or break) to form a new linear polypeptide with different N-terminal and C-terminal from the original polypeptide.

Importantly, the circularly permutated peptide provides optimized ends for fusion with other peptides while retaining the biological activity of the original peptide. If the new end blocks the essential region of the initial peptide, it may lose its activity. Similarly, if connecting the original end will destroy the activity, the circularly permutated peptide cannot retain its biological activity. Therefore, there are two requirements for the production of an active circulating permutated protein: 1) connecting the end of the initial polypeptide does not destroy its biological activity; 2) there must be at least one "opening site" in the initial polypeptide, where a new end can be formed without destroying the region critical to its folding and biological activity.

Therefore, in general, in the natural folding state (original protein), the original N-terminal and C-terminal of the candidate peptide for circularly permutation are very close, for example, the distance between the N-terminal and C-terminal of the original protein is less than or equal to 20 Å.

For fusing with the desired polypeptide fusion partner and reducing the length of the required linker, the location of the new terminal is advantageous in geometry, structure and function (relative to the natural terminal).

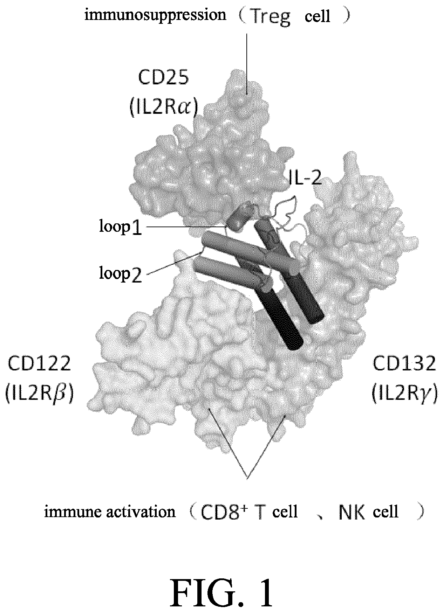

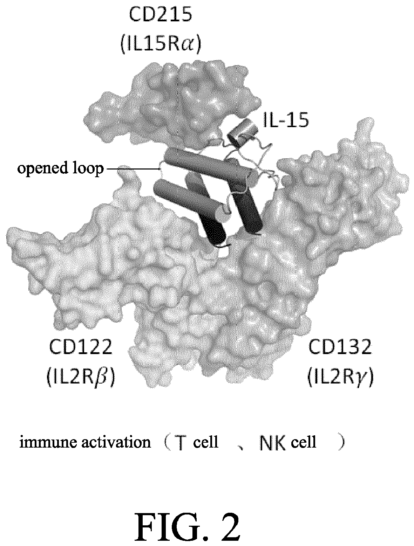

Fig. 1 shows the structure of IL-2, wherein loop 1 and loop 2 are examples of positions where new ends are formed. Fig. 2 shows the structure of IL-15, wherein the "opened loop" is an example of the position where a new end is formed.

In some embodiments, to perform circular permutation on IL-2, the engineering design is conducted for the recombinant construct, where the natural N-terminal and C-terminal of IL-2 are linked via a linker, and the cyclic molecule is opened between amino acid residues A93-R103 or N50-L56 to form a linear molecule with new N-terminal and C-terminal. In some embodiments, new N-terminal is formed at amino acid residue R103, new C-terminal is formed at amino acid residue A93 and amino acid residue Q94-P102 is deleted, i.e., IL-2 (R103/A93). In some embodiments, the circularly permutated IL-2 is IL-2 (L56/N50), IL-2 (L56/K55) or IL-2 (N53/K52). In some embodiments, new N-terminal is formed at amino acid residue N97 and new C-terminal is formed at amino acid residue K96, i.e., IL-2 (N97/K96).

In some embodiments, the circularly permutated IL-2 comprises an amino acid sequence of SEQ ID NO: 2, 3, 4 or 5.

In some embodiments, to perform circular permutation on IL-15, the engineering design is conducted for the recombinant construct, where the natural N-terminal and C-terminal of IL-15 are linked via a linker to form a cyclic molecule, and the cyclic molecule is opened between amino acid residence S102-A105 to form a linear molecule with new N-terminal and C-terminal. For example, new C-terminal is formed at amino acid residues S102, G103 or D104, and new N-terminal is formed at G103, D104, A105 or S106. In some embodiments, the circularly permutated IL-15 is IL-15 (G103/S102), IL-15 (D104/S102), IL-15 (A105/S102), IL-15 (S106/S102), IL-15 (S106/G103), IL-15 (A105/G103), IL-15 (D104/G103), IL-15 (A105/D104), IL-15 (S106/D104), IL-15 (S106/A105).

In a preferable embodiment, the length of the linker used to link N-terminal and C-terminal of the initial polypeptide is related to the distance between N-terminal and C-terminal in the original protein. In some embodiments, the linker used to link N-terminal and C-terminal of the initial polypeptide has a length of 1-10 amino acids, such as 1, 2, 3, 4, 5, 6, 7, 8, 9 or 10 amino acids. In some embodiments, the linker has a length of more than 10 amino acids. Preferably, a linker of 3 amino acids e.g., GSG is used to link N-terminal and C-terminal of natural IL-2; a linker of 4 amino acids e.g., GGGG (SEQ ID NO: 17) is used to link N-terminal and C-terminal of natural IL-15.

Those skilled in the art will understand that other modifications can be made to the initial polypeptide, such as amino acid substitution. In some embodiments, residue T23 of natural IL-2 (i.e., T2 of SEQ ID NO: 1) is substituted to A. In some embodiments, residue C145 of natural IL-2 (i.e., C124 of SEQ ID NO: 1) is substituted to S.

In some embodiments, the circularly permutated IL-2 comprises an amino acid sequence of SEQ ID NO: 2, 3, 4 or 5.

In some embodiments, the circularly permutated IL-15 comprises an amino acid sequence of SEQ ID NO: 7.

In some embodiments, the circularly permutated IL-2 comprises an amino acid sequence of SEQ ID NO: 23.

In some embodiments, the circularly permutated IL-15 comprises an amino acid sequence of SEQ ID NO: 24.

III. Fusion polypeptide

The fusion polypeptide according to the present disclosure comprises a carrier protein and a polypeptide of interest, wherein the carrier protein has a plurality of helical domains linked with a loop, the polypeptide of interest is inserted into the loop of the carrier protein, the carrier protein shields a site of interest on the polypeptide of interest, thereby blocking the accessibility of the site.

The inventors found that when inserting the polypeptide of interest into a loop of the carrier protein, by selecting an appropriate insertion site, adjusting the linker (such as its sequence and/or length) between the polypeptide of interest and the carrier protein, deleting one or more amino acids in the inserted loop, deleting one or more amino acids at N-terminal and/or C-terminal of the polypeptide of interest, or any combination thereof, the dihedral angle in the peptide chain can be employed to control the relative position of the carrier protein and the polypeptide of interest, such that the carrier protein can shield the site of interest.

In some embodiments, the polypeptide of interest is derived from a cytokine of the four α-helical bundle cytokine family, including but not limited to IL-2, IL-4, IL6, IL-7, IL-9, IL15 and IL21. Preferably, the polypeptide of interest is a cytokine of the circularly permutated four α-helical bundle cytokine family. In a preferable embodiment, the polypeptide of interest is circularly permutated IL-2 or IL-15.

Natural IL-2 has binding sites for CD25, CD122 and CD132 (known as IL-2 receptors α, β and γ, respectively). IL-2 can not only bind to CD25/CD122/CD132 trimer to activate T regulatory cells (Treg) expressing the trimer and inhibit immune response, but also bind to CD122/CD132 dimer to activate immune cells (such as CD8+ memory T cell and NK cell) expressing the dimer and stimulate its proliferation. The binding of CD25 to IL-2 will change the conformation of IL-2 and increase its affinity for CD122/CD132 dimer.

Accordingly, in some embodiments, the polypeptide of interest is circularly permutated IL-2, the site of interest is CD25 binding site, and the fusion polypeptide has an activity comparable to or improved over natural IL-2, such as that of activating the JAK1/JAK3 and STAT3/STAT5 signal pathways.

IL-15 can also activate the immune response, which plays an important role in the differentiation and proliferation of T cells and NK cells, as well as the development of dendritic cells.

The working mechanism of IL-15 is similar to that of IL-2. It activates the downstream signal pathways (JAK1/JAK3 and STAT3/STAT5) by binding to CD122/CD132 receptor dimer, but its α receptor is different from that of IL-2 and is a unique IL-15R α (CD215). After binding to IL-15Rα, IL-15 binds to CD122/CD132 with high affinity, while in the case of not binding to IL-15Rα, IL-15binds to CD122/CD132 with medium affinity.

Accordingly, in some embodiments, the polypeptide of interest is circularly permutated IL-15, the site of interest is CD215 binding site, and the fusion polypeptide has an activity comparable to or improved over natural IL-15, such as that of activating the JAK1/JAK3 and STAT3/STAT5 signal pathways.

Preferably, the carrier protein suitable herein can bind to a newborn Fc receptor (FcRn) (such as albumin), or the carrier protein can bind to a protein which can bind to FcRn (such as albumin binding protein)

Human serum albumin (HSA) is one of the most stable proteins with the highest content (35-50 g/L) in human serum, accounting for half of the proteins in serum. Its main role is to transport substances (such as hormones, fatty acids, or the like), maintain pH and osmotic pressure. HSA herein comprises wild type HSA and modified HSA

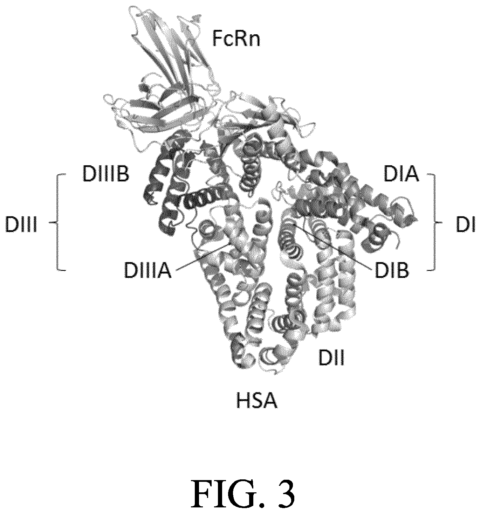

HSA is a whole α helix protein with a molecular weight of 66.5 kDa. It is composed of three similar domains (DI, DII, DIII) forming a "heart" structure (Fig. 3). HSA can bind to human FcRn under acidic condition (pH<6.5), and then be recovered to the cell surface and released back into the blood, preventing HSA from entering lysosome and being degraded. FcRn mainly bind to DIII and a part of DI.

The precursor sequence of HSA is as shown in SEQ ID NO: 16 (Uniprot P02768). All positions involving HSA herein are numbered with reference to SEQ ID NO: 16. In the precursor sequence of HSA, residues 1-24 is a signal peptide, and natural HSA comprises residues 25-609 of SEQ ID NO: 16.

In some embodiments, the carrier protein is HSA. Preferably, the insertion of the polypeptide of interest does not affect the binding of HSA and FcRn. In some embodiments, the polypeptide of interest is inserted into the loop of HSA, wherein the loop is selected from the group consisting of loops at D56-L66, A92-P96, D129-E131, Q170-A172, K281-L283, V293-L305, E311-S312, E321-A322, A362-D365, L398-E400, K439-R445, E465-D471, P537-E542, A561-T566, and preferably loops at D56-L66, V293-L305 and A362-D365. In some embodiments, the insertion site of the polypeptide of interest is amino acid residue D56, A300, C361 or A362 of HSA.

The relative position between the carrier protein and the polypeptide of interest by deleting one or more amino acids in the loop of the carrier protein such as HSA, or the amino acids in the loop of the carrier protein may not be deleted.

HSA can also be modified to improve its properties. For example, a free cysteines in the wild type HSA can be replaced with another amino acid, such as serine. In some embodiments, the HSA comprises an amino acid substitution C58S.

In some embodiments, the carrier protein has at least 70%, 80%, 90%, 95%, 96%, 97%, 98% or 99% sequence identity to SEQ ID NO: 16, and has an identical or similar structure as wild type HSA.

In some embodiments, N-terminal and C-terminal of the polypeptide of interest is linked to the carrier protein via a linker. In some embodiments, N-terminal of the polypeptide of interest is linked to the carrier protein via a linker, C-terminal of the polypeptide of interest is directly linked to the carrier protein. In some embodiments, N-terminal of the polypeptide of interest is directly linked to the carrier protein, C-terminal of the polypeptide of interest is linked to the carrier protein via a linker. In some embodiments, N-terminal and C-terminal are directly linked to the carrier protein. In some embodiments, the linker has 1, 2, 3, 4, 5, 6, 7, 8, 9, 10, 11, 12, 13, 14, 15, 16, 17, 18, 19, 20 or more amino acids. In some embodiments, the linker is selected from the group consisting of a helical linker, or a flexible linker (e.g., GS linker and polyglycine linker ).

In some embodiments, the fusion polypeptide according to the present disclosure comprises a carrier protein HSA and a circularly permutated IL-2, wherein the circularly permuted IL-2 comprises four α-helical bundles of, from N-terminal to C-terminal, H3, H4, H1 and H2, or H4, H1, H2 and H3; and wherein the circularly permuted IL-2 is inserted into a loop of the HSA, the loop is selected from the group consisting of loops at D56-L66, A92-P96, D129-E131, Q170-A172, K281-L283, V293-L305, E311-S312, E321-A322, A362-D365, L398-E400, K439-R445, E465-D471, P537-E542 and A561-T566, the positions are numbered by reference to SEQ ID NO: 16, the HSA shields the CD25 binding site of the circularly permuted IL-2, thereby blocking the accessibility of the site. In some embodiments, the circularly permutated IL-2 comprises an amino acid sequence of SEQ ID NO: 2, 3, 4 or 5. In some embodiments, the loop is selected from the group consisting of loops at D56-L66, V293-L305 and A362-D365 of HSA. In some embodiments, the insertion site of the circularly permuted IL-2 is selected from the group consisting of D56, A300, C361 and A362 of HSA.

In some embodiments, the circularly permutated IL-2 comprises an amino acid sequence of SEQ ID NO: 2. In some embodiments, the loop is a loop at A362-D365 of HSA. Preferably, the insertion site is C361 of HSA. In some embodiments, N-terminal of the circularly permutated IL-2 is linked to C361 of HSA via a linker EAAAKAEAAA (SEQ ID NO: 19), C-terminal is linked to D365 of HSA via a linker GS, and residues 362-364 of HSA are deleted.

In some embodiments, the circularly permutated IL-2 comprises an amino acid sequence of SEQ ID NO: 4. In some embodiments, the loop is a loop at D56-L66 of HSA. Preferably, the insertion site is D56 of HSA. In some embodiments, N-terminal of the circularly permutated IL-2 is linked to D56 of HSA via a linker AAAAAK (SEQ ID NO: 20), C-terminal is directly linked to E57 of HSA.

In some embodiments, the circularly permutated IL-2 comprises an amino acid sequence of SEQ ID NO: 5. In some embodiments, the loop is a ring at V293-L305 of HSA. Preferably, the insertion site is A300 of HSA. In some embodiments, N-terminal of the circularly permutated IL-2 is directly linked to A300 of HSA, C-terminal is linked to D301 of HSA via a linker G.

In some embodiments, the circularly permutated IL-2 consists of an amino acid sequence SEQ ID NO: 4. In some embodiments, the loop is a loop at A362-D365 of HSA. Preferably, the insertion site is A362 of HSA. In some embodiments, N-terminal of the circularly permutated IL-2 is directly linked to A362 of HSA, C-terminal is directly linked to A363 of HSA.

In some embodiments, provided is a fusion polypeptide, comprising a carrier protein HSA and a circularly permutated IL-15, wherein the circularly permutated IL-15 comprises four α-helical bundles of, from N-terminal to C-terminal, H3, H4, H1 and H2; and wherein the circularly permutated IL-15 is inserted into a loop of the HSA, the loop is selected from the group consisting of loops at D56-L66, A92-P96, D129-E131, Q170-A172, K281-L283, V293-L305, E311-SS312, E321-A322, A362-D365, L398-E400, K439-R445, E465-D471, P537-E542 and A561-T566, the positions are renumbered by reference to SEQ ID NO: 16, the HSA shields the CD215 binding site of the circularly permutated IL-15, thereby blocking the accessibility of the site. In some embodiments, the circularly permutated IL-15 comprises an amino acid sequence of SEQ ID NO: 7.

In some embodiments, the fusion polypeptide according to the present disclosure comprises an amino acid sequence of one of SEQ ID NO: 8-11. In some embodiments, the fusion polypeptide according to the present disclosure comprises an amino acid sequence which has at least 70%, 80%, 90%, 95%, 96%, 97%, 98% or 99% sequence identity to one of SEQ ID NO: 8-11, wherein the fusion polypeptide comprises natural CD25 binding site and has an activity comparable to or improved over natural IL-2, such as that of activating the JAK1/JAK3 and STAT3/STAT5 signal pathways.

In some embodiments, the fusion polypeptide according to the present disclosure has more lasting immune stimulating effect. In some embodiments, the fusion polypeptide according to the present disclosure has higher safety.

In some embodiments, as compared to natural IL-2, the fusion polypeptide according to the present disclosure has a half-life increased by 10%, 20%, 30%, 40%, 50%, 60%, 70%, 80%, 90%, 100%, 150%, 200%, 300%, 400%, 500%, 600%, 700%, 800%, 900%, 1000% or more. In some embodiments, as compared to natural IL-15, the fusion polypeptide according to the present disclosure has a half-life increased by 10%, 20%, 30%, 40%, 50%, 60%, 70%, 80%, 90%, 100%, 150%, 200%, 300%, 400%, 500%, 600%, 700%, 800%, 900%, 1000% or more.

## IV. Polypeptide dimer

The polypeptide dimer according to the present disclosure comprises a first polypeptide and a second polypeptide,

wherein the first polypeptide comprises a first dimerization domain and a polypeptide of interest, wherein the polypeptide of interest is located at a first end of the first dimerization domain,
wherein the second polypeptide comprises a second dimerization domain and a binding domain, wherein the binding domain is located at a first end of the second dimerization domain,
wherein the first polypeptide and the second polypeptide form a dimmer with the first dimerization domain and the second dimerization domain, and wherein the first end of the first dimerization domain is adjacent to the first end of the second dimerization domain, the binding domain is capable of binding to the site of interest on the polypeptide of interest.

In some embodiments, the polypeptide of interest is derived from a cytokine of the four $\alpha$-helical bundle cytokine family, including but not limited to IL-2, IL-4, IL6, IL-7, IL-9, IL15 and IL21. Preferably, the polypeptide of interest is a cytokine of the circularly permutated four $\alpha$-helical bundle cytokine family. In a preferable embodiment, the polypeptide of interest is circularly permutated IL-2 or IL-15, wherein the circular permutation is as described above.

Native IL-2 has binding sites for CD25, CD122 and CD132 (known as IL-2 receptors $\alpha$, $\beta$ and $\gamma$, respectively). IL-2 can not only bind to CD25/CD122/CD132 trimer to activate T regulatory cells (Treg) expressing the trimer and inhibit immune response, but also bind to CD122/CD132 dimer to activate immune cells (such as CD8+ memory T cell and NK cell) expressing the dimer and stimulate its proliferation. The binding of CD25 and IL-2 will change the conformation of IL-2 and increase its affinity for CD122/CD132 dimer.

Accordingly, in some embodiments, the polypeptide of interest is circularly permutated IL-2, the site of interest is CD25 binding site, the binding domain is extracellular domain of CD25, and the polypeptide dimer has an activity comparable to or improved over natural IL-2, such as that of activating the JAK1/JAK3 and STAT3/STAT5 signal pathways.

IL-15 can also activate the immune response, which plays an important role in the differentiation and proliferation of T cells and NK cells, as well as the development of dendritic cells. The working mechanism of IL-15 is similar to that of IL-2. It activates the downstream signal pathways (JAK1/JAK3 and STAT3/STATS) by binding to CD122/CD132 receptor dimer, but its $\alpha$ receptor is different from that of IL-2 and is a unique EL-15R$\alpha$ (CD215). After binding to IL-15R$\alpha$, IL-15 binds to CD122/CD132 with high affinity, while in the case of not binding to IL-15R$\alpha$, IL-15 binds to CD122/CD132 with medium affinity.

Accordingly, in some embodiments, the polypeptide of interest is circularly permutated IL-15, the site of interest is CD215 binding site, the binding domain is extracellular domain of CD215, and the polypeptide dimer has an activity comparable to or improved over natural IL-15, such as that of activating the JAK1/JAK3 and STAT3/STAT5 signal pathways.

In the polypeptide dimer according to the present disclosure, the site of interest and the binding domain are located in different (first and second) polypeptides. The first dimerization domain and second dimerization domain form a dimmer, making the site of interest close to the binding domain. The first dimerization domain and second dimerization domain form a dimmer by for example but not limited to covalent bonding, hydrogen bonding, electrostatic interaction and/or van der Waals force, preferably covalent bonding.

In some embodiments, the first dimerization domain and second dimerization domain form a dimmer by disulfide bond. In some embodiments, the first dimerization domain and second dimerization domain comprise heavy chain constant region CH2 and CH3 of immunoglobulin (Ig), such as human Ig (e.g., human IgG1). In some embodiments, the first dimerization domain and second dimerization domain form Fc region of human IgG1.

Various mutations can be introduced in the Fc segment to achieve different functions, for example, to increase the affinity of Fc and FcRn under acidic condition; reduce or increase the affinity of Fc with different Fc $\gamma$ receptors and C1q to weaken or enhance antibody dependent cytotoxicity (ADCC), antibody dependent cell phagocytosis (ADCP), complement dependent cytotoxicity (CDC) or the like. In some embodiments, Fc region in the polypeptide dimer according to the present disclosure comprises Fc silent mutations, such as L234A+L235A+P329G, so as to reduce ADCC, ADCP and CDC, wherein the amino acid positions in the Fc region are numbered according to IMGT EU numbering rule (http://www.imgt.org/IMGTScientificChart/Numbering/Hu_IGHGnber.html).

In some embodiments, the Fc region is a homodimer, that is, the first dimerization domain has a sequence identical to that of the second dimerization domain. In some embodiments, the Fc region is a heterodimer, that is, the first dimerization domain has a sequence different to that of the second dimerization domain. In some embodiments, a knob-in-hole modification is made to the Fc region. For example, a mutation T366Y (the 1st generation knob-in-hole) or S354C+T366W (the 2nd generation knob-in-hole) is introduced into a polypeptide chain to form a knob chain; and Y407T (the 1st generation knob-in-hole) or Y349C+T366S+L368A+Y407V (the 2nd generation knob-in-hole) is introduced into another polypeptide chain to form a hole chain, where the numbering rule is as described above.

In some embodiments, the first polypeptide comprises a knob chain and the second polypeptide comprises a hole chain. In some embodiments, the first polypeptide comprises a hole chain, and the second polypeptide comprises a knob chain.

In some embodiments, the knob chain comprises an amino acid sequence of SEQ ID NO: 23 or an amino acid sequence which has at least 70%, 80%, 90%, 95%, 96%, 97%, 98% or 99% sequence identity to SEQ ID NO: 23. In some embodiments, the hole chain comprises an amino acid sequence of SEQ ID NO: 24 or an amino acid sequence which has at least 70%, 80%, 90%, 95%, 96%, 97%, 98% or 99% sequence identity to SEQ ID NO: 24.

In some embodiments, the first dimerization domain comprises an amino acid sequence of SEQ ID NO: 23 or an amino acid sequence which has at least 70%, 80%, 90%, 95%, 96%, 97%, 98% or 99% sequence identity to SEQ ID NO: 23, and the second dimerization domain comprises an amino acid sequence of SEQ ID NO: 24 or an amino acid sequence which has at least 70%, 80%, 90%, 95%, 96%, 97%, 98% or 99% sequence identity to SEQ ID NO: 24; or the first dimerization domain comprises an amino acid sequence of SEQ ID NO: 24 or an amino acid sequence which has at least 70%, 80%, 90%, 95%, 96%, 97%, 98% or 99% sequence identity to SEQ ID NO: 24, and the second dimerization domain comprises an amino acid sequence of SEQ ID NO: 23 or an amino acid sequence which has at least 70%, 80%, 90%, 95%, 96%, 97%, 98% or 99% sequence identity to SEQ ID NO: 23.

In some embodiments involving immunoglobulin Fc region, the polypeptide of interest is located at C-terminal of the first dimerization domain (i.e., the first chain of Fc region), and the binding domain is located at C-terminal of the second dimerization domain (i.e., the second chain of Fc region). In some embodiments involving immunoglobulin Fc region, the polypeptide of interest is located at N-terminal of the first dimerization domain (i.e., the first chain of Fc region), and the binding domain is located at N-terminal of the second dimerization domain (i.e., the second chain of Fc region).

In some embodiments, the polypeptide of interest is linked to the first dimerization domain via a linker and the binding domain is linked to the second dimerization domain via a linker. In some embodiments, the linker is a flexible linker. In some embodiments, the linker has 1, 2, 3, 4, 5, 6, 7, 8, 9, 10, 11, 12, 13, 14, 15, 16, 17, 18, 19, 20 or more amino acids. In some embodiments, the linker has 1-20, 2-18, 3-16, 5-15, 6-12 or 8-10 amino acids. In some embodiments, the linker has an amino acid sequence of GGGGSGGGGS (SEQ ID NO: 30).

In some embodiments, the polypeptide dimer according to the present disclosure comprises a first polypeptide and a second polypeptide, wherein the first polypeptide comprises a first chain of Fc region of human IgG1 and a circularly permutated IL-2, the circularly permutated IL-2 is linked to C-terminal of the first chain of Fc region of human IgG1, wherein the second polypeptide comprises a second chain of Fc region of human IgG1 and a CD25 extracellular domain, the CD25 extracellular domain is linked to C-terminal of Fc region of human IgG1, and wherein the circularly permutated IL-2 is as described above, for example the circularly permutated IL-2 consists of an amino acid sequence of SEQ ID NO: 21. In some embodiments, the first chain consists of an amino acid of SEQ ID NO: 23, and the second chain consists of an amino acid sequence of SEQ ID NO: 24; or the first chain consists of an amino acid of SEQ ID NO: 24, and the second chain consists of an amino acid sequence of SEQ ID NO: 23.

In some embodiments, the polypeptide dimer according to the present disclosure comprises a first polypeptide and a second polypeptide, wherein the first polypeptide comprises a first chain of Fc region of human IgG1 and a circularly permutated IL-15, the circularly permutated IL-15 is linked to C-terminal of the first chain of Fc region of human IgG1, wherein the second polypeptide comprises a second chain of Fc region of human IgG1 and a CD215 extracellular domain, the CD215 extracellular domain is linked to C-terminal of the second chain of Fc region of human IgG1, and wherein the circularly permutated IL-15 is as described above, for example, the circularly permutated IL-15 comprises an amino acid sequence of SEQ ID NO: 22. In some embodiments, the first chain comprises an amino acid sequence of SEQ ID NO: 23 and the second chain comprises an amino acid of SEQ ID NO: 24; or the first chain comprises an amino acid sequence of SEQ ID NO: 24 and the second chain comprises an amino acid of SEQ ID NO: 23.

Those skilled in the art will understand that the amino acid sequence of a circularly permutated polypeptide can be modified without reducing its biological activity. Such modifications are well-known to those skilled in the art, and include the addition of a residue, such as methionine added at the amino end to provide an initiation site, or the addition of an additional amino acid at either end to protect the protein from damage by exopeptidase.

Those skilled in the art will understand that other modifications can be made. For example, amino acid substitutions, such as conservative substitutions, can be made without affecting protein activity. Alternatively, the unnecessary region of the molecule can be reduced or completely eliminated. Therefore, in the regions where the molecule *per se* is not involved in the molecular activity, they can be eliminated or replaced by shorter fragments, which are only used to maintain the correct spatial relationship between the active components of the molecule.

In some embodiments, the circularly permutated IL-2 comprises an amino acid sequence which has at least 70%, 80%, 90%, 95%, 96%, 97%, 98% or 99% sequence identity to SEQ ID NO: 21, wherein the first polypeptide comprises the natural CD25 binding site, and the polypeptide dimer has an activity comparable to or improved over natural IL-2.

In some embodiments, the circularly permutated IL-15 comprises an amino acid sequence which has at least 70%, 80%, 90%, 95%, 96%, 97%, 98% or 99% sequence identity to SEQ ID NO: 22, wherein the first polypeptide comprises the natural CD215 binding site, and the polypeptide dimer has an activity comparable to or improved over natural IL-15.

In some embodiments, the first polypeptide comprises an amino acid sequence of SEQ ID NO: 25, the second polypeptide comprises an amino acid sequence of SEQ ID NO: 26. In some embodiments, the first polypeptide comprises an amino acid sequence which has at 70%, 80%, 90%, 95%, 96%, 97%, 98% or 99% sequence identity to SEQ ID NO: 25, the second polypeptide comprises an amino acid sequence which has at 70%, 80%, 90%, 95%, 96%, 97%, 98% or 99% sequence identity to SEQ ID NO: 26, wherein the first polypeptide comprises the natural CD25 binding site, and the polypeptide dimer has an activity comparable to or improved over natural IL-2.

In some embodiments, the first polypeptide comprises an amino acid sequence of SEQ ID NO: 27, the second polypeptide comprises an amino acid sequence of SEQ ID NO: 28. In some embodiments, the first polypeptide comprises an amino acid sequence which has at 70%, 80%, 90%, 95%, 96%, 97%, 98% or 99% sequence identity to SEQ ID NO: 27, the second polypeptide comprises an amino acid sequence which has at 70%, 80%, 90%, 95%, 96%, 97%, 98% or 99% sequence identity to SEQ ID NO: 28, wherein the first polypeptide comprises the natural CD25 binding site, and the polypeptide dimer has an activity comparable to or improved over natural IL-2.

In some embodiments, as compared to natural IL-2, the polypeptide dimer according to the present disclosure has a half-life increased by 10%, 20%, 30%, 40%, 50%, 60%, 70%, 80%, 90%, 100%, 150%, 200% or more.

In some embodiments, as compared to natural IL-15, the polypeptide dimer according to the present disclosure has a half-life increased by 10%, 20%, 30%, 40%, 50%, 60%, 70%, 80%, 90%, 100%, 150%, 200% or more.

## V. Expression of the fusion polypeptide and polypeptide dimer

To express the fusion polypeptide according to the present disclosure, provided is also a polynucleotide encoding the fusion polypeptide according to the present disclosure.

To express the polypeptide dimer according to the present disclosure, provided is also a polynucleotide encoding the polypeptide dimer according to the present disclosure. In some embodiments, the first polypeptide and second polypeptide are encoded by a single polynucleotide. In some embodiments, the first polypeptide and second polypeptide are encoded by different polynucleotides.

The nucleic acid molecules of all or part of the nucleic acid sequences according to the present disclosure can be isolated by polymerase chain reaction (PCR), using oligonucleotide primers designed and synthesized based on the sequence information contained in the sequences.

The polynucleotides according to the present disclosure can be amplified using cDNA, mRNA or genomic DNA as templates and appropriate oligonucleotide primers according to standard PCR amplification technology. The nucleic acid thus amplified can be cloned into a suitable vector and characterized by DNA sequence analysis.

The polynucleotide according to the present disclosure can be prepared by standard synthesis technology, such as using an automatic DNA synthesizer.

Provided is also a complementary chain of nucleic acid molecule described herein. The nucleic acid molecule complementary to another nucleotide sequence is a molecule sufficiently complementary to the nucleotide sequence, such that it can hybridize with the nucleotide sequence to form a stable double strand. Of course, the polynucleotide according to the present disclosure does not comprise the polynucleotide which only hybridizes only with the poly A sequence (such as 3'-end poly(A) of mRNA) or that hybrids with a section of complementary poly T (or U) residues.

Also provided is a vector comprising the polynucleotide according to the present disclosure, preferably an expression vector. Further provided is a host cell comprising the polynucleotide or carrier (preferably, an expression vector) according to the present disclosure. In some embodiments, the polynucleotide according to the present disclosure is incorporated into the genome of the host cell. In some annotations, the polynucleotide according to the present disclosure is not incorporated into the genome of the host cell.

In some embodiments, the first polypeptide and second polypeptide of the fusion polypeptide according to the present disclosure are express by a single expression vector. In some embodiments, the first polypeptide and second polypeptide of the fusion polypeptide according to the present disclosure are express by different vectors.

The selection of expression vectors is related to the host cell used to express the fusion polypeptide or polypeptide dimer. The host cells which can be used to express the fusion polypeptide according to the present disclosure comprise but not limited to bacteria (including E. coli.), yeast, insect cells and mammalian cells, such as COS, CHO, HeLa and 293-6E cells. Expression vectors suitable for various host cells are known in the art. For example, expression vectors suitable for bacteria comprise but not limited to pET vectors (such as pET-28a, pET-30a, pET-32a and pET-40a or the like), pEX vectors (such as pEX-1), pGH112, pUC118 and pEZZ18; expression vectors suitable for yeast comprise but not limited to pESP vectors (such as pESP-1, pESP-2, pESP-3 or the like), pDR196, pHiSi, p53his, pSH47 and pYCP211; expression vectors suitable for insect cells comprise but not limited to pCoBlast, pIEX/Bac-3, pIEXBac-c-EGFP-4, pFastBac1-His-C, pIEXBac-c-EGFP-3, pFastBac1-GST-N and pIEXBac-c-EGFP-2; expression vectors suitable for mammalian cells comprise but not limited to pCMVInt, pGL4.23, pX334, pX458, pBiFC-CC155, pDP4rs, pDC312 and pcDNA.

In some inclusions, the host cell is 293-6E cell. In some annotations, the expression vector is pcDNA3.4.

The precursor polypeptide comprises a signal peptide which facilitates the secretion and/or processing of the expressed polypeptide. Therefore, in some embodiments, the polynucleotide according to the present disclosure further comprises a sequence encoding the signal peptide. In some embodiments, the signal peptide comprises an amino acid sequence of METDTLLLWVLLLWVPGSTG (SEQ ID NO: 18).

The polynucleotides or vectors according to the present disclosure can be transferred (transfected) into selected host cells with methods known in the art, such as calcium chloride transformation and calcium phosphate treatment for *E. coli*, electroporation, lipid transfection amine treatment or PEI treatment for mammalian cells. The cells transformed by the vectors can be selected according to the antibiotic resistance genes (such as amp, gpt, neo and hyg genes) in the vectors.

After expression, the recombinant fusion protein can be purified according to standard procedures in the field, including ammonium sulfate precipitation, affinity chromatography, column chromatography using ion or hydrophobic resins, gel electrophoresis or the like. A substantially pure composition with a purity of at least about 90 to 95% is preferable, and a purity of 98 to 99% or higher is most preferably used for pharmaceutical purposes.

In order to facilitate purification, the fusion polypeptide according to the present disclosure can also include a tag sequence, including but not limited to His6 tag, FLAG tag, or the like.

In some embodiments, the fusion polypeptide according to the present disclosure comprises an amino acid sequence of one of SEQ ID NO: 12-15. In some embodiments, the fusion polypeptide according to the present disclosure comprises an amino acid sequence which has at least 70%, 80%, 90%, 95%, 96%, 97%, 98% or 99% sequence identity to one of SEQ ID NO: 12-15, wherein the fusion polypeptide comprises the natural CD25 binding site and has an activity comparable to or improved over natural IL-2, such as that of activating the JAK1/JAK3 and STAT3/STAT5 signal pathways.

In some embodiments, the polynucleotide or vector such as expression vector according to the present disclosure encodes an amino acid sequence of SEQ ID NO: 31 and an amino acid sequence of SEQ ID NO: 32. In some embodiments, the polynucleotide or vector such as expression vector according to the present disclosure encodes an amino acid sequence which has at least 70%, 80%, 90%, 95%, 96%, 97%, 98% or 99% sequence identity to SEQ ID NO: 31 and an amino acid sequence which has at least 70%, 80%, 90%, 95%, 96%, 97%, 98% or 99% sequence identity to SEQ ID NO: 32, wherein the expressed first polypeptide comprises the natural CD25 binding site and the polypeptide dimer has an activity comparable to or improved over natural IL-2, such as that of activating the JAK1/JAK3 and STAT3/STAT5 signal pathways.

In some embodiments, the polynucleotide or vector such as expression vector according to the present disclosure encodes an amino acid sequence of SEQ ID NO: 33 and an amino acid sequence of SEQ ID NO: 34. In some embodiments, the polynucleotide or vector such as expression vector according to the present disclosure encodes an amino acid sequence which has at least 70%, 80%, 90%, 95%, 96%, 97%, 98% or 99% sequence identity to SEQ ID NO: 33 and an amino acid sequence which has at least 70%, 80%, 90%, 95%, 96%, 97%, 98% or 99% sequence identity to SEQ ID NO: 34, wherein the expressed first polypeptide comprises the natural CD25 binding site and the polypeptide dimer has an activity comparable to or improved over natural IL-2, such as that of activating the JAK1/JAK3 and STAT3/STAT5 signal pathways.

VI. Pharmaceutical composition

Provided is a pharmaceutical composition comprising the fusion polypeptide according to the present disclosure. In an embodiment, the pharmaceutical composition comprises the fusion polypeptide according to the present disclosure and at least one pharmaceutically acceptable carrier. The fusion polypeptide according to the present disclosure can be combined with a pharmaceutically acceptable carrier according to known methods to prepare the pharmaceutical composition.

Provided is also a pharmaceutical composition comprising the polypeptide dimer according to the present disclosure. In an embodiment, the pharmaceutical composition comprises the polypeptide dimer according to the present disclosure and at least one pharmaceutically acceptable carrier. The polypeptide dimer according to the present disclosure can be combined with a pharmaceutically acceptable carrier according to known methods to prepare the pharmaceutical composition.

The pharmaceutical acceptable carrier includes but not limited to solvent, emulsifier, buffer, stabilizer or the like. The solvent comprises water, aqueous solution and nonaqueous solvent (such as vegetable oil).

The pharmaceutical composition according to the present disclosure can be applied by any suitable route, including subcutaneous, intramuscular, intraarticular, intravenous, intradermal, intraperitoneal, intranasal, intracranial, parenteral administration. Preferably, the pharmaceutical composition according to the present disclosure is administered intravenously. It should be understood that the administration route may vary with the therapeutic agent, the condition and age of the recipient, and the disease being treated.

The pharmaceutical composition according to the present disclosure may be a solution or a lyophilized preparation.

In some embodiments, the pharmaceutical composition according to the present disclosure is provided in the form of freeze-dried powders for rehydration before administration. The pharmaceutical composition according to the present disclosure may also be provided in a liquid form, which can be directly applied to the patient. In some compositions, the composition is provided in a pre-filled syringe as a liquid.

In some embodiments, the composition according to the present disclosure is encapsulated in liposomes. In some embodiments, liposomes can be coated with flexible water-soluble polymers, which can avoid being taken up by organs of mononuclear phagocytic system, mainly liver and spleen. Hydrophilic polymers suitable for coating liposomes compries but not limited to, PEG, polyvinylpyrrolidone, polyvinylmethyl ether, polymethyloxazoline, polyethyloxazoline, polyhydroxypropyloxazoline, polyhydroxypropylmethylacrylamide, polymethylacrylamide, polydimethylacrylamide, polyhydroxypropylmethacrylate, polyoxyethylacrylate, hydroxymethylcellulose hydroxyethylamide, hydrophilic polyvinyl alcohol or the like.

The pharmaceutical composition according to the present disclosure can be administered once or multiple times according to the dosage and frequency required and tolerated by the the patient. In any case, the applied pharmaceutical composition should provide sufficient protein according to the present disclosure to effectively treat the patient.

VII. Treatment application

Provided further is a method for treat a disease, such as that related to immunosuppression with the fusion polypeptide according to the present disclosure.

In some embodiments, provided is a method for treating a cancer, comprising administering a subject in need thereof the fusion polypeptide or the pharmaceutical composition according to the present disclosure in an effective amount. The cancer comprises but not limited to lung cancer, liver cancer, kidney cancer, head and neck cancer, colorectal cancer, gastric cancer, nasopharyngeal cancer, glioma, melanoma and osteosarcoma.

In some embodiments, provided is a method for activating an immune cell or improving proliferation of an immune cell, comprising administering a subject in need thereof the fusion polypeptide or the pharmaceutical composition according to the present disclosure in an effective amount. In some embodiments, the immune cell is T cell or NK cell.

In some embodiments, provided is use of the fusion polypeptide or the pharmaceutical composition according to the present disclosure for the manufacture of a medicament for treating a cancer. The cancer comprises but not limited to lung cancer, liver cancer, kidney cancer, head and neck cancer, colorectal cancer, gastric cancer, nasopharyngeal cancer, glioma, melanoma and osteosarcoma.

In some embodiments, provided is use of the fusion polypeptide or the pharmaceutical composition according to the present disclosure for the manufacture of a medicament for activating an immune cell or improving proliferation of an immune cell. In some embodiments, the immune cell is T cell or NK cell.

Provided further is a method for treat a disease, such as that related to immunosuppression with the polypeptide dimer according to the present disclosure.

In some embodiments, provided is a method for treating a cancer, comprising administering a subject in need thereof the polypeptide dimer or the pharmaceutical composition according to the present disclosure in an effective amount. The cancer comprises but not limited to lung cancer, liver cancer, kidney cancer, head and neck cancer, colorectal cancer, gastric cancer, nasopharyngeal cancer, glioma, melanoma and osteosarcoma.

In some embodiments, provided is a method for activating an immune cell or improving proliferation of an immune cell, comprising administering a subject in need thereof the polypeptide dimer or the pharmaceutical composition according to the present disclosure in an effective amount. In some embodiments, the immune cell is T cell or NK cell.

In some embodiments, provided is use of the polypeptide dimer or the pharmaceutical composition according to the present disclosure for the manufacture of a medicament for treating a cancer. The cancer comprises but not limited to lung cancer, liver cancer, kidney cancer, head and neck cancer, colorectal cancer, gastric cancer, nasopharyngeal cancer, glioma, melanoma and osteosarcoma.

In some embodiments, provided is use of the polypeptide dimer or the pharmaceutical composition according to the present disclosure for the manufacture of a medicament for activating an immune cell or improving proliferation of an immune cell. In some embodiments, the immune cell is T cell or NK cell.

Embodiments

P1. A polypeptide dimer, comprising a first polypeptide and a second polypeptide,

wherein the first polypeptide comprises a first dimerization domain and a polypeptide of interest, wherein the polypeptide of interest is located at the first end of the first dimerization domain,
wherein the second polypeptide comprises a second dimerization domain and a binding domain, wherein the binding domain is located at the first end of the second dimerization domain,
wherein the first polypeptide forms a dimer with the second polypeptide through the first dimerization domain and the second dimerization domain, and wherein the first end of the first dimerization domain is adjacent to the first end of the second dimerization domain, the binding domain is capable of binding to the site of interest on the polypeptide of interest.

P2. The polypeptide dimer according to Embodiment P1, wherein the polypeptide of interest is derived from a cytokine of the four α-helical bundle cytokine family, the cytokine comprises four α-helix bundles of, from N-terminal to C-terminal, helical bundle 1 (H1), helical bundle 2 (H2), helical bundle 3 (H3) and helical bundle 4 (H4).

P3. The polypeptide dimer according to Embodiment P2, wherein the polypeptide of interest is a cytokine of the circularly permutated four α-helical bundle cytokine family, comprising four α-helical bundles of, from N-terminal to C-terminal, H2, H3, H4 and H1; H3, H4, H1 and H2; or H4, H1, H2 and H3.

P4. The polypeptide dimer according to Embodiment P3, wherein the amino acid in the circularly permutated cytokine corresponding to N-terminal of the natural cytokine is linked to the amino acid corresponding to C-terminal of the natural cytokine via a linker.

P5. The polypeptide dimer according to Embodiment P4, wherein the linker is a GS linker or a polyglycine linker having a length of 1-10 amino acids.

P6. The polypeptide dimer according to any one of Embodiments P1-P5, wherein the polypeptide of interest is a circularly permutated IL-2 or a circularly permutated IL-15.

P7. The polypeptide dimer according to Embodiment P6, wherein the circularly permutated IL-2 comprises four α-helical bundles of, from N-terminal to C-terminal, H3, H4, H1 and H2, or H4, H1, H2 and H3.

P8. The polypeptide dimer according to Embodiment P7, wherein the circularly permutated IL-2 comprises an amino acid sequence of SEQ ID NO: 21.

P9. The polypeptide dimer according to Embodiment P7 or P8, wherein the site of interest is a CD25 binding site, the binding domain is an extracellular domain of CD25.

P10. The polypeptide dimer according to Embodiment P6, wherein the circularly permutated IL-15 comprises four α-helical bundles of, from N-terminal to C-terminal, H3, H4, H1 and H2.

P11. The polypeptide dimer according to Embodiment P10, wherein the circularly permutated IL-15 comprises an amino acid sequence of SEQ ID NO: 4.

P12. The polypeptide dimer according to Embodiment P10 or P11, wherein the site of interest is a CD215 binding site, the binding domain is an extracellular domain of CD215.

P13. The polypeptide dimer according to any one of Embodiments P1-P12, wherein the first and second dimerization domains comprise heavy chain constant region CH2 and CH3 of immunoglobulin (Ig).

P14. The polypeptide dimer according to Embodiment P13, wherein the Ig is human Ig.

P15. The polypeptide dimer according to Embodiment P14, wherein the Ig is human IgG1.

P16. The polypeptide dimer according to any one of Embodiments P1-P15, wherein the first dimerization domain forms Fc region of the human IgG1 with the second dimerization domain.

P17. The polypeptide dimer according to any one of Embodiments P1-P16, wherein the first dimerization domain comprises an amino acid sequence of SEQ ID NO: 21, and the second dimerization domain comprises an amino acid sequence of SEQ ID NO: 22; or the first dimerization domain comprises an amino acid sequence of SEQ ID NO: 22, and the second dimerization domain comprises an amino acid sequence of SEQ ID NO: 21.

P18. The polypeptide dimer according to any one of Embodiments P13-P17, wherein the first end of the first dimerization domain is C-terminal, and the first end of the second dimerization domain is C-terminal.

P19. A polypeptide dimer, comprising a first polypeptide and a second polypeptide,

wherein the first polypeptide comprises a first chain of the Fc region of the human IgG1 and a circularly permutated IL-2, the circularly permutated IL-2 is linked to C-terminal of the first chain of the Fc region of the human IgG1,
wherein the second polypeptide comprises a second chain of the Fc region of the human IgG1 and a CD25 extracellular domain, the CD25 extracellular domain is linked to C-terminal of the second chain of the Fc region of the human IgG1, and

wherein the circularly permutated IL-2 comprises four α-helical bundles of, from N-terminal to C-terminal, H3, H4, H1 and H2; or H4, H1, H2 and H3.

P20. The polypeptide dimer according to Embodiment P19, wherein the circularly permutated IL-2 comprises an amino acid sequence of SEQ ID NO: 2.

P21. A polypeptide dimer, comprising a first polypeptide and a second polypeptide,

wherein the first polypeptide comprises a first chain of the Fc region of the human IgG1 and a circularly permutated IL-15, the circularly permutated IL-15 is linked to C-terminal of the first chain of the Fc region of the human IgG1, wherein the second polypeptide comprises a second chain of the Fc region of the human IgG1 and a CD215 extracellular domain, the CD215 extracellular domain is linked to C-terminal of the second chain of the Fc region of the human IgG1, and

wherein the circularly permutated IL-15 comprises four α-helical bundles of, from N-terminal to C-terminal, H3, H4, H1 and H2.

P22. The polypeptide dimer according to Embodiment P21, wherein the circularly permutated IL-15 comprises an amino acid sequence of SEQ ID NO: 22.

P23. The polypeptide dimer according to any one of Embodiments P19-P22, wherein the first chain comprises an amino acid sequence of SEQ ID NO: 23 and the second chain comprises an amino acid sequence of SEQ ID NO: 24; or the first chain comprises an amino acid sequence of SEQ ID NO: 24 and the second chain comprises an amino acid sequence of SEQ ID NO: 23.

P24. A pharmaceutical composition, comprising the polypeptide dimer according to any one of Embodiments P1-P23.

P25. Use of the polypeptide dimer according to any one of Embodiments P1-P23 for the manufacture of a medicament for treating a cancer.

P26. Use of the polypeptide dimer according to any one of Embodiments P1-P23 for the manufacture of a medicament for activating an immune cell or improving proliferation of an immune cell.

P27. The use according to Embodiment P26, wherein the immune cell is T cell or NK cell.

P28. One or more isolated polynucleotides, encoding the polypeptide dimer according to any one of Embodiments P1-P23.

P29. One or more expression vectors, comprising the polynucleotide according to Embodiment P28.

P30. A host cell, comprising the polynucleotide according to Embodiment P28 or the vector according to Embodiment P29.

### Examples

Through the following examples, those skilled in the art will have a better understanding of the present disclosure. It should be understood that the examples are provided only for illustration rather than limitation to the scope of the present disclosure. Unless otherwise specified, the experimental methods used herein are conventional methods, and the specific procedures of gene cloning can be found in Sambrook, et al. (Molecular Cloning: A Laboratory Manual, 2nd, ed. Cold Spring Harbor Laboratory, Cold Spring Harbor Laboratory Press, Cold Spring Harbor, N.Y, 1989).

### Example 1 Construction of expression vector

The construction of expression vector was completed by Nanjing GenScript Biotech Corp., including synthesis of nucleic acid encoding amino acid sequences of SEQ ID NO: 12-15 and 31-34, additionally containing the nucleotide sequence encoding signal peptide of SEQ ID NO: 18; construction of the nucleic acid into the mammalian cell expression vector pcDNA 3.4 by molecular cloning method, and amplification and purification of the plasmids.

### Example 2 Expression of fusion polypeptide and polypeptide dimer

In this Example, 293-6E cells were transfected with nucleic acid encoding fusion polypeptide for eukaryotic expression. The expression and purification of fusion polypeptide was completed by Nanjing GenScript Biotech Corp. The procedures were as follows:

- 293-6E cells were cultured in serum free FreeStyle™ 293 Expression medium (Thermo Fisher Scientific, Carlsbad, CA, USA) in Erlenmeyer conical flask (Corning Inc., Acton, MA), where the culture was performed on vibrating incubator (VWR Scientific, Chester, PA) at 37°C and 5% $CO_2$.

- The cells were diluted to the proper density one day prior to transfection.
- On the day of transfection, the plasmid mixture (the mass ratio of the plasmid encoding the first polypeptide and that encoding the second polypeptide was 1: 1) and the transfection reagents (such as Polyetherimide, PEI) were mixed in a suitable ratio (such as 1:3 mass ratio) in the culture medium, and then added into the cell culture medium for transfection for secretion and expression of the target protein.
- After six days, the cell survival rate was about 50%-80%. The supernatant was obtained by centrifugation, which contained the target protein (the expressed polypeptide did not contain the signal peptide).
- After filtration with 0.22μm membrane, the supernatant was loaded into HisTrap™ FF Crude 5ml (GE, catalog No. 17-5286-01) chromatographic column at a rate of 3 ml/min. After washing and elution, the purified protein was collected and stored in phosphate buffer solution (PBS pH 7.2) through buffer exchange.

The molecular weight, purity and sequence coverage of the purified protein were evaluated by SDS-PAGE, Western blot (results not shown), high performance liquid chromatography with molecular sieve (SEC-HPLC) and liquid chromatography mass spectrometry (LC-MS). The primary antibody used in immunoblotting was mouse anti-His tag antibody (GenScript, catalog No. A00186) or mouse anti-FLAG antibody (GenScript, catalog No. A00187), the secondary antibody was horseradish peroxidase modified goat anti mouse IgG antibody (GenScript, catalog No. A00160).

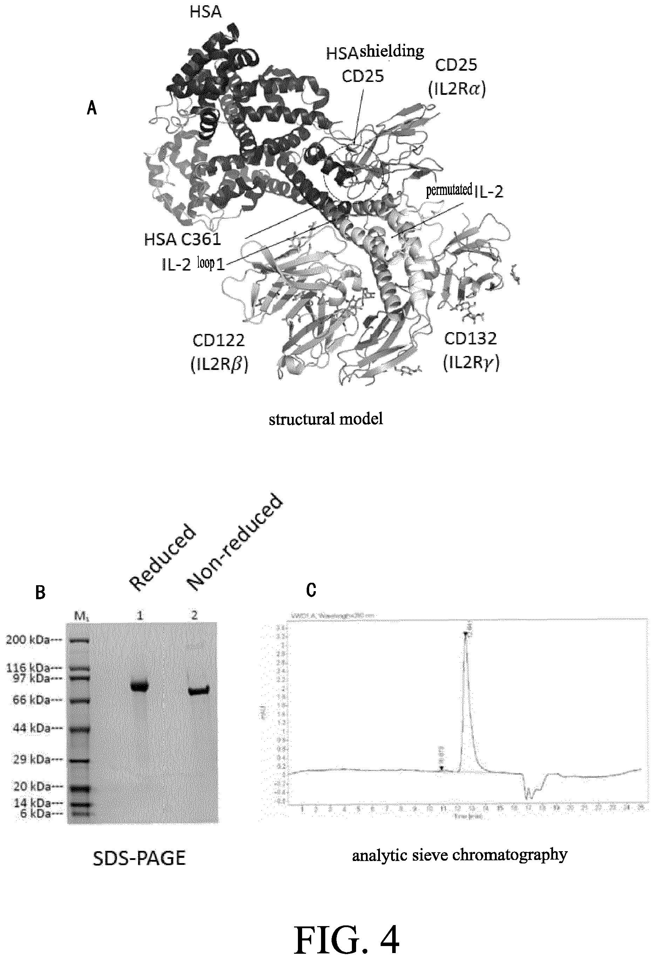

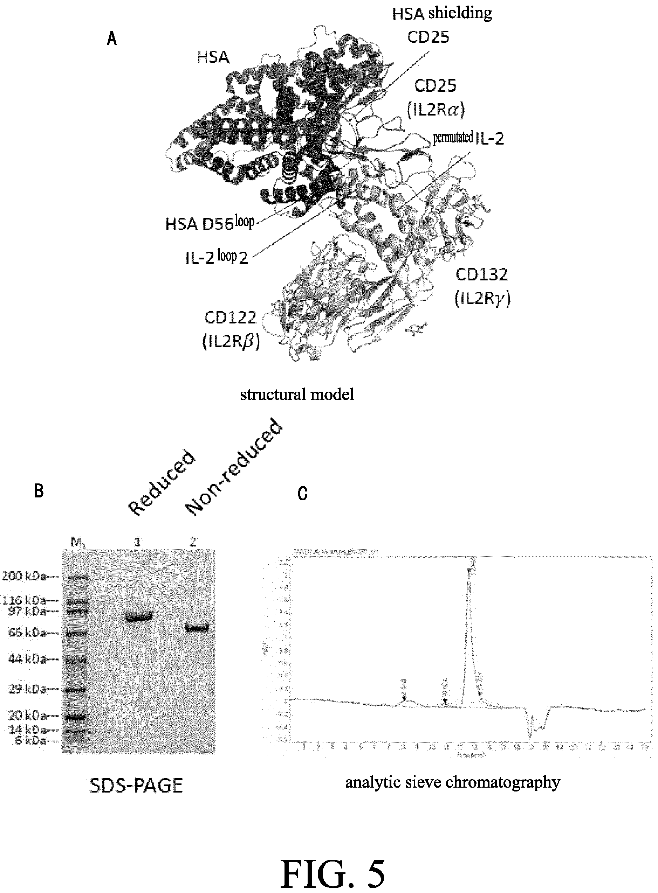

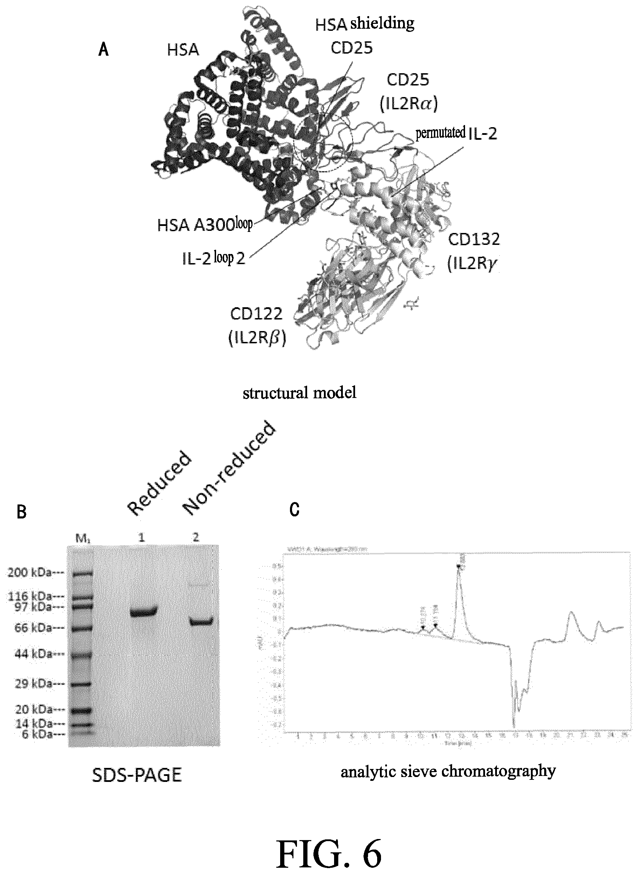

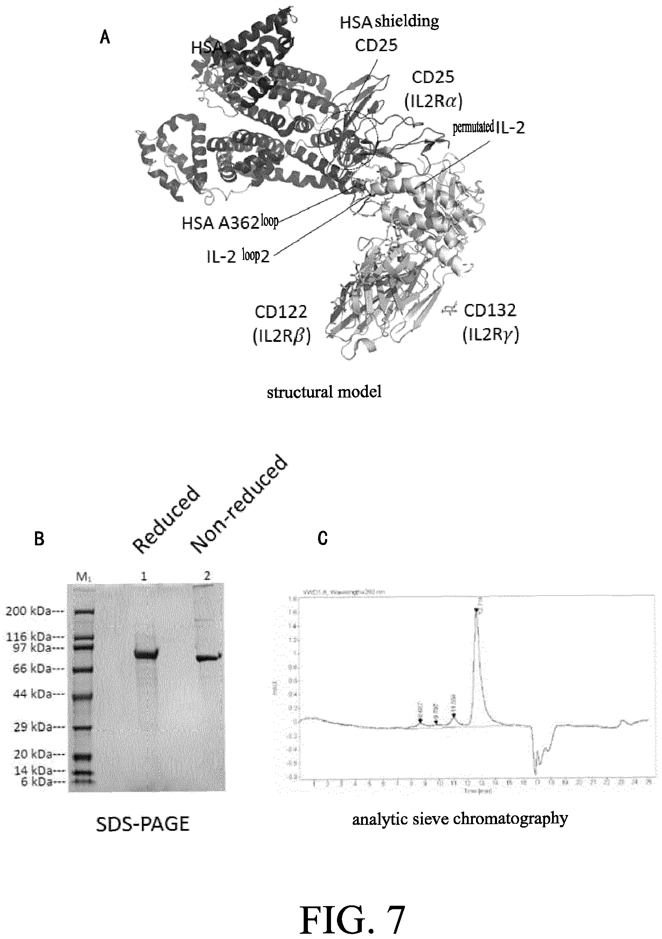

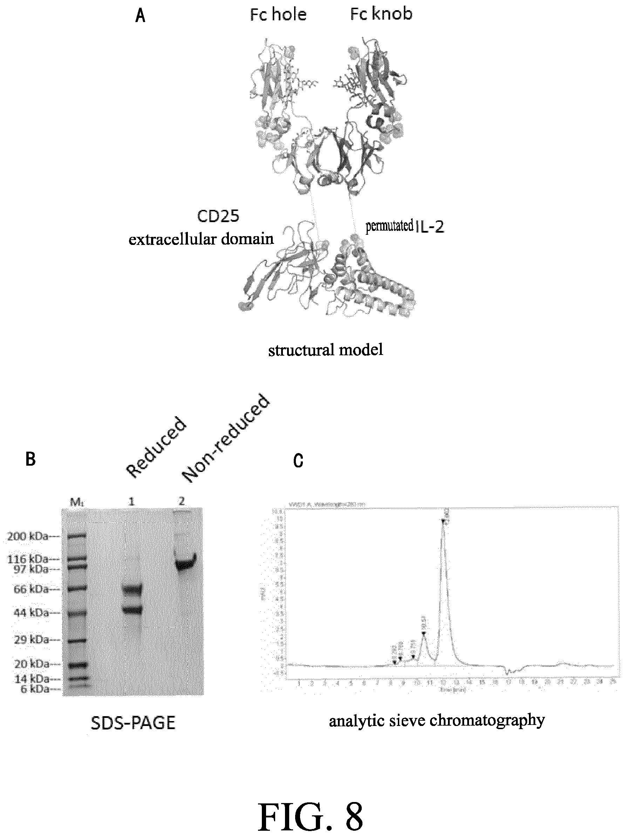

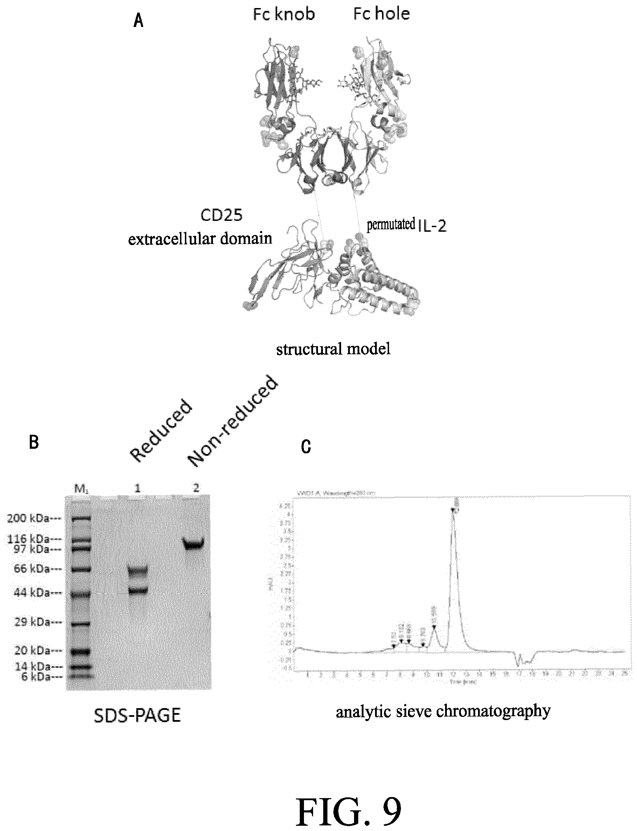

The results were shown in Fig. 4-9. Fig. 4: loop 1 of IL-2 was opened to form circularly permutated IL-2, and the insertion site was at C361 of HSA(SEQ ID NO: 12). Fig. 5: loop 2 of IL-2 was opened to form circularly permutated IL-2, and the insertion site was at D56 of HSA (SEQ ID NO: 13). Fig. 6: loop 2 of IL-2 was opened to form circularly permutated IL-2, and the insertion site was at A300 of HSA (SEQ ID NO: 14). Fig 7: loop 1 of IL-2 was opened to form circularly permutated IL-2, and the insertion site was in A362 of HSA (SEQ ID NO: 15). Fig. 8: circularly permutated IL-2 was fused with knob chain of knob-in-hole modified human IgG1 Fc region (SEQ ID NO: 31), extracellular domain of CD25 was fused with hole chain of knob-in-hole modified human IgG1 Fc region (SEQ ID NO: 32). Fig. 9: circularly permutated IL-2 was fused with hole chain of knob-in-hole modified human IgG1 Fc region (SEQ ID NO: 33), extracellular domain of CD25 was fused with knob chain of knob-in-hole modified human IgG1 Fc region (SEQ ID NO: 34). In Fig In 4-7, panel A showed the structural model of CD25 binding site of IL-2 shielded by HSA, panel B showed the SDS-PAGE results of reduced and non-reduced fusion polypeptides, and panel C showed the molecular sieve analysis results. In Fig In 8 and 9, panel A showed the structural model of the polypeptide dimer according to the present disclosure, panel B showed the SDS-PAGE results of reduced and non-reduced polypeptide dimers, and panel C showed the molecular sieve analysis results.

As shown in Fig As shown in 4-7, four fusion polypeptides achieved high expression levels. SDS-PAGE and SEC-HPLC showed high purity and high homogeneity. High sequence coverage was detected by LC-MS, confirming the integrity and correctness of the purified fusion polypeptides.

As shown in Fig. 8 and 9, both polypeptide dimers achieved high expression levels. SDS-PAGE and SEC-HPLC showed high purity and high homogeneity. High sequence coverage was detected by LC-MS, confirming the integrity and correctness of the purified dimers.

**Example 3 Detection of activities of fusion polypeptide and polypeptide dimer**

In this example, HEK-Blue™ IL-2 cell line (InvivoGen) was used to detect the activity of these fusion proteins in activating downstream signal pathways at the cell level.

The HEK-Blue™ IL-2 cell line was constructed by stable transfection of the genes encoding the molecules required for IL-2 signal pathways in human embryonic kidney 293 cells (HEK-293), the molecules including CD25, CD122 and CD132 receptor molecules, human JAK3 kinase and transcription factor STATS, together with the secretory embryonic alkaline phosphatase (SEAP) reporter gene regulated by STATS. When the cell line was stimulated by IL-2, it will activate the downstream signal pathways to activate STATS and upregulate the secretion and expression of SEAP.

The above detection was completed by Nanjing Topoxin Biotechnology Co., Ltd. The procedures were as follows:

1. Experiment preparation

1.1. Preparation of QUANTI Blue solution: QB reagent and QB buffer were thawed at room temperature, to which was added 98 mL of sterile water, after blending, the mixture was divided into 10 mL/tube, and stored in dark at -20°C.

1.2. Inactivation of serum: 45 mL of serum (FBS) was placed into a 50 mL centrifuge tube, with heat inactivation in 56°C water bath for 30 min (mixing every 10 min), and was stored at 4°C for further use.

### 1.3. Culture medium

Growth medium: DMEM + 10% FBS + 1% PS + 100 $\mu$g/mL Normocin + 1 $\mu$g/ml puromycin + 1X HEK-Blue™ CLR Selection; Testing medium: DMEM+10% FBS (Inactivated)+1% PS+100 $\mu$g/mL Normocin; Frozen medium: DMEM+20% FBS+10% DMSO.

### 2. Cell culture, passage and preservation

2.1. The cells were placed in the growth medium, and cultured with passage in an incubator at 37°C, 5% $CO_2$;

2.2. When the cell confluence reached 70-80% and the cells were under good condition, the supernatant was discarded and the growth medium preheated in 37°C water bath was used to resuspend the cells. After cell counting, 200×g centrifugation was conducted for 5 min, and the supernatant was discarded. The cells was suspended with 4°C precooled frozen medium, and the cell density was adjusted to 5-7×10$^6$ cells/mL, 1 mL per tube. The tubes were put into the programing cooling box, and stored overnight at -80°C with liquid nitrogen for long-term storage.

### 3. Verification with HEK-Blue IL-2 system

3.1. Preparation of HEK-Blue IL-2 cell suspension: the cells were rinsed gently twice with preheated DPBS and resuspended with preheated DPBS to form a single cell suspension. After cell counting, 200×g centrifugation was conducted for 5 min. The cells were suspended with preheated testing medium, with the cell density of 2.8×10$^5$ cells/mL.

3.2. Samples of human IL-2 protein (positive control, AcroBiosystems, catalog No. IL2-H4113) and HSA (negative control) or human IgG1 Fc segment (negative control, AcroBiosystems, catalog No. FCC-H5214) were prepared with serial gradient dilution as follows: initial concentration 650pM, 5-fold dilution, 8 gradients.

3.3. 20 $\mu$L of sample of serial gradient dilution and 180 $\mu$L of cell suspension were added to each well of the culture plate (three replicates for each sample), the plate was cultured in incubator at 37°C, 5% $CO_2$ for 20-24 h.

3.4. Supernatant of each culture was added into 96-well plate at 20 $\mu$L/well, and the QUANTI-Blue solution thawed and recovered to room temperature was added at 180 $\mu$L/well, at 37°C, with incubation for 1-3 h. read with A microplate reader was used to read the absorbance at OD 620-655 nm, and analyze the data.

### 4. Detection of fusion polypeptide activity

The procedures in step 3 were empolyed to detect the activity of fusion polypeptide and polypeptide dimer prepared in Example 2, wherein each fusion polypeptide was also prepared into a series of gradient dilution samples according to the procedures in step 3.2.

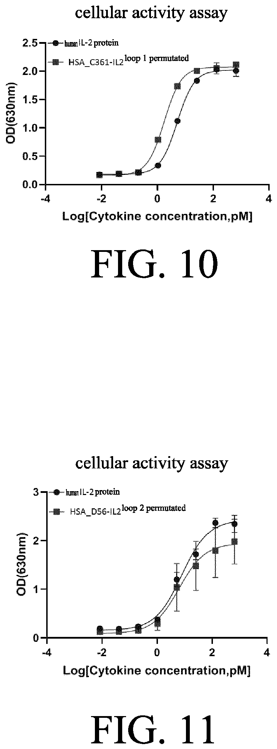

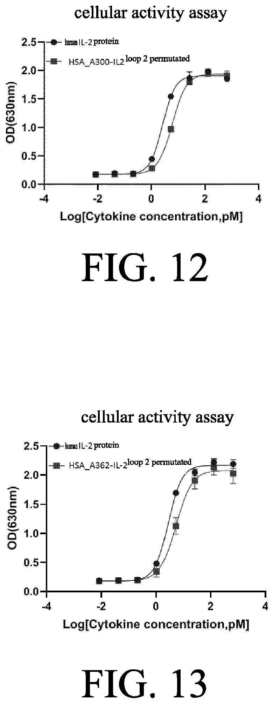

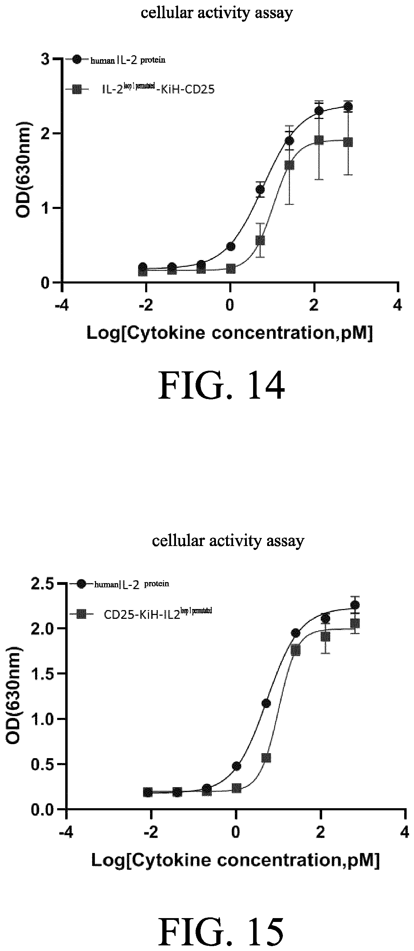

The results were shown in Fig 10-15. Fig. 10: loop 1 of IL-2 was opened to form circularly permutated IL-2, and the insertion site was at C361 of HSA (SEQ ID NO: 12). Fig. 11: loop 2 of IL-2 was opened to form circularly permutated IL-2, and the insertion site was at D56 of HSA (SEQ ID NO: 13). Fig. 12: loop 2 of IL-2 was opened to form circularly permutated IL-2, and the insertion site was at A300 of HSA(SEQ ID NO: 14). Fig 13: loop 1 of IL-2 was opened to form circularly permutated IL-2, and the insertion site was in A362 of HSA (SEQ ID NO: 15). Fig. 14: the first polypeptide comprised circularly permutated IL-2, which was fused with knob chain of knob-in-hole modified human IgG1 Fc region (SEQ ID NO: 31), the second polypeptide comprised extracellular domain of CD25, which was fused with hole chain of knob-in-hole modified human IgG1 Fc region (SEQ ID NO: 32). Fig. 15: the first polypeptide comprised circularly permutated IL-2, which was fused with hole chain of knob-in-hole modified human IgG1 Fc region (SEQ ID NO: 33), the second polypeptide comprised extracellular domain of CD25, which was fused with knob chain of knob-in-hole modified human IgG1 Fc region (SEQ ID NO: 34).

The comparisons between the concentration for 50% of maximal effect (EC50) of each fusion polypeptides as detected and EC50 of human IL-2 were shown in Tables 1-6 below.

Table 1 Comparison between fusion polypeptide of SEQ ID NO: 12 and human IL-2

|  | Human IL-2 | Fusion polypeptide of SEQ ID NO: 12 |
| --- | --- | --- |
| EC50 (pM) | 5.123 | 1.750 |

Table 2 Comparison between fusion polypeptide of SEQ ID NO: 13 and human IL-2

|  | Human IL-2 | Fusion polypeptide of SEQ ID NO: 13 |
|---|---|---|
| EC50 (pM) | 7.934 | 6.356 |

Table 3 Comparison between fusion polypeptide of SEQ ID NO: 14 and human IL-2

|  | Human IL-2 | Fusion polypeptide of SEQ ID NO: 14 |
|---|---|---|
| EC50 (pM) | 2.583 | 5.859 |

Table 4 Comparison between fusion polypeptide of SEQ ID NO: 15 and human IL-2

|  | Human IL-2 | Fusion polypeptide of SEQ ID NO: 15 |
|---|---|---|
| EC50 (pM) | 2.755 | 5.253 |

Table 5 Comparison between polypeptide dimer 1 (SEQ ID NO: 31+ SEQ ID NO: 32) and human IL-2

|  | Human IL-2 | Polypeptide dimer 1 |
|---|---|---|
| EC50 (pM) | 6.117 | 10.83 |

Table 6 Comparison between polypeptide dimer 2 (SEQ ID NO: 33+ SEQ ID NO: 34) and human IL-2

|  | Human IL-2 | Polypeptide dimer 2 |
|---|---|---|
| EC50 (pM) | 5.406 | 10.17 |

The results showed that the fusion polypeptide according to the present disclosure showed an activity comparable to or improved over wildtype IL-2, while the HSA or Fc region fragment did not show the corresponding response (results not shown).

**Example 4 Detection of interaction between fusion polypeptides and IL-2 receptor**

The interaction between the fusion polypeptide or the polypeptide dimer according to the present disorder and IL-2 receptor was measured using a high-throughput molecular interaction instrument based on the principle of surface plasmon resonance imaging (SPRi).

1. The following receptor proteins or combinations of receptor proteins were immobilized on the surface of carboxyl chip:

□ CD25;
□ CD122;
□ CD25 + CD122;
□ CD122 + CD132; and
CD25+CD122+CD132.

2. The wildtype IL-2 protein solutions at gradient concentration were used as the mobile phase, and the SPRi interaction experiment was conducted according to the manufacturer's instructions to measure the binding signal and affinity.

3. The fusion polypeptide or the polypeptide dimer solutions according to the present disclosure prepared following the results of step 2 at gradient concentrations were used as the mobile phase, and the SPRi interaction experiment was conducted according to the manufacturer's instructions to measure the binding signal and affinity.

**Example 5 Detection of *in vivo* activity of fusion polypeptide**

Activities of five selected fusion polypeptide or polypeptide dimer molecules were measured (see Table 7 below) in B16F10 melanoma mouse model for activating the immune system to kill tumors, and the infiltration of CD8+ T cells in tumor tissue was observed. The experiments were entrusted to Jiangsu Jichu Biotechnology Co., Ltd.

Table 7

| Code of molecule | Name of molecule | SEQ ID NO: |
|---|---|---|
| ZJ-1 | IL-2$^{\text{loop1 permutated}}$-KiH-CD25 | 31+32 |
| ZJ-2 | HSA_C361-IL2$^{\text{loop1 permutated}}$ | 12 |
| ZJ-6 | HSA_D56-IL2$^{\text{loop2 permutated}}$ | 13 |
| ZJ-12 | HSA_A300-IL2$^{\text{loop2 permutated}}$ | 14 |
| ZJ-15 | HSA_A362-IL-2$^{\text{loop2 permutated}}$ | 15 |

5.1. Determination of anti-tumor activity in *in vivo* model

B16F10 melanoma cells were cultured in DMEM (Dulbecco's Modified Eagle's Medium) medium + 10% (v/v) fetal bovine serum (FBS).

C57BL/6 mice were fed adaptively for a week, B16F10 melanoma cells ($1\times10^6$/animal) were injected subcutaneously, and the mice were kept for 7 days to allow the tumor to grow. The mice with tumors were divided into six groups (9 mice in each group) and injected with the fusion polypeptide or polypeptide dimer molecules and blank control (PBS), respectively in the tail vein. The injection amounts of the fusion polypeptide or polypeptide dimer molecules was 2 mg/kg body weight, with injection once every nine days, three times in total (D0, D9 and D18). The body weight and tumor size (length and width) of each mouse were measured every three days to calculate the tumor volume. The survived mice were sacrificed 27 days after the first injection, and the tumors were taken for photos and embedded in wax for pathological sections for subsequent immunohistochemical experiments.

5.2. Immunohistochemical detection of CD8 expression in tumor sections

a). The slices were subjected to dewaxing and hydration; xylene treatment for 3 times, 5 min each time; anhydrous ethanol treatment for 2 times, 5 min each time; 95% ethanol treatment for 2 times, 5 min each time; distilled water treatment for 2 times, 5 min each time.

b). After thermal remediation with citric acid antigen remediation method, the samples were cooled to room temperature, and washed with PBS for 3 times, 5 min each time.

c). The immunostaining blocking solution was used for blocking at room temperature for 15 min, and then the blocking solution was removed without washing. The anti-CD 8 primary antibody (dilution ratio: 1:2000) was added for incubation at room temperature for 1 h.

d). The samples were washed with PBS for three times, 5 min each time; and incubated with secondary antibody for 1 h.

e). The samples were washed with PBS for three times, 5 min each time; and developed with DAB, and the end point of development was determined by microscopic observation.

f). After development, hematoxylin was used for re-staining for 2 min, and tap water was used for returning blue. The samples were subjected to dehydration with ethanol of gradient concentration, made transparent with xylene, and sealed with neutral gum.

g). Image ProPlus software was used to conduct semiquantitative analysis on immunohistochemical staining results:

Average optical density (IOD/area) = cumulative optical density value/measured area of dyed area.

The higher the average optical density, the stronger the positive.

5.3. Experimental results

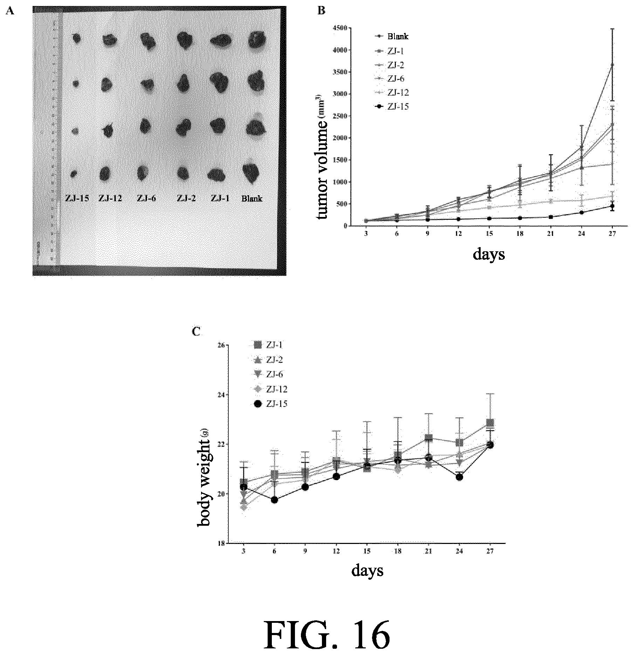

As shown in Fig. 16A and B, at the dose of 2 mg/kg, in all of the selected five fusion polypeptide or polypeptide dimer showed anti-tumor activities in mouse melanoma models, among which ZJ-15 showed the strongest activity, and nearly no tumor growth. Compared with the data reported in the prior art (Charych et al., Clin Canc Res 2016), the fusion polypeptide or polypeptide dimer molecules according to the present disclosure (especially, ZJ-15 and ZJ-12) showed significantly superior antitumor activity *in vivo* over wildtype IL-2 with the same animal tumor model, dose, administration mode and frequency.

As shown in Fig. 16C, when the fusion polypeptide or polypeptide dimer molecule according to the present disclosure were administered to mice at a dose of 2 mg/kg (relatively high for cytokine), the body weights of mice still steadily increased. Such results showed that the fusion polypeptide or polypeptide dimer molecules according to the present disclosure had good tolerance potential *in vivo*, proving the safety of the fusion polypeptide or polypeptide dimer molecules according to the present disclosure.

The expression of CD8 was observed by immunohistochemical staining of tumor tissue sections to detect the infiltration of CD8+T cells (killing T cells) in tumors.

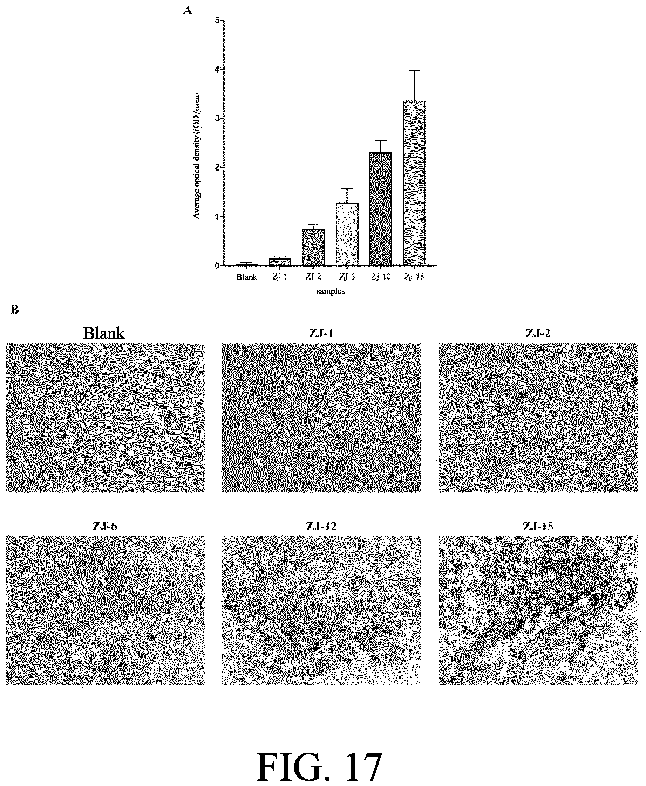

As shown in Fig. 17A, the degree of invasion of CD8+T cells induced by these molecules was highly correlated with their anti-tumor activities (see Fig. 16A and B). The higher the degree of infiltration of CD8+T cells, the more inhibition to tumor growth. This was consistent with the working mechanism of the fusion polypeptide or polypeptide dimer molecules according to the present disclosure expected by the inventor, that is, the fusion polypeptide or polypeptide dimer molecules according to the present disclosure can activate the body's own immune system to kill tumor tissues in a better way. Fig. 17B showed the immunohistochemical staining photos of some representative tumor sections, which more intuitively showed the infiltration of CD8+T cells in tumor tissues under the interventions of different molecules, in which blue represented the nucleus, and brown represented the CD8 expression signal. It can be clearly observed that, for example, ZJ-15 molecule induce a large number of CD8+ T cells to enter tumor tissues for killing and inhibition.

**Claims**

1.   A fusion polypeptide, comprising a carrier protein and a polypeptide of interest, wherein the carrier protein has a plurality of helical domains linked with loops, the polypeptide of interest is inserted into a loop of the carrier protein, the carrier protein shields a site of interest on the polypeptide of interest, thereby blocking the accessibility of the site.

2.   The fusion polypeptide according to claim 1, wherein the polypeptide of interest is derived from a cytokine of the four $\alpha$-helical bundle cytokine family, the cytokine comprises four $\alpha$-helix bundles of, from N-terminal to C-terminal, helical bundle 1 (H1), helical bundle 2 (H2), helical bundle 3 (H3) and helical bundle 4 (H4).

3.   The fusion polypeptide according to claim 1, wherein the polypeptide of interest is a circularly permutated cytokine of the four $\alpha$-helical bundle cytokine family, comprising four $\alpha$-helical bundles of, from N-terminal to C-terminal, H2, H3, H4 and H1; H3, H4, H1 and H2; or H4, H1, H2 and H3.

4.   The fusion polypeptide according to claim 3, wherein the amino acid in the circularly permutated cytokine corresponding to N-terminal of the uncircularly permutated cytokine is linked to the amino acid corresponding to C-terminal of the uncircularly permutated cytokine via a linker.

5.   The fusion polypeptide according to claim 4, wherein the linker is a GS linker or a polyglycine linker having a length of 1-10 amino acids.

6.   The fusion polypeptide according to any one of claims 1-5, wherein the polypeptide of interest is selected from the group consisting of a circularly permutated IL-2 and a circularly permutated IL-15.

7.   The fusion polypeptide according to claim 6, wherein the circularly permutated IL-2 comprises four $\alpha$-helical bundles of, from N-terminal to C-terminal, H3, H4, H1 and H2, or H4, H1, H2 and H3.

8.   The fusion polypeptide according to claim 7, wherein the circularly permutated IL-2 comprises an amino acid sequence of SEQ ID NO: 2, 3, 4 or 5.

9.   The fusion polypeptide according to claim 7 or 8, wherein the site of interest is a CD25 binding site.

**10.** The fusion polypeptide according to claim 6, wherein the circularly permutated IL-15 comprises four α-helical bundles of, from N-terminal to C-terminal, H3, H4, H1 and H2.

**11.** The fusion polypeptide according to claim 10, wherein the circularly permutated IL-15 comprises an amino acid sequence of SEQ ID NO: 7.

**12.** The fusion polypeptide according to claim 10 or 11, wherein the site of interest is a CD215 binding site.

**13.** The fusion polypeptide according to any one of claims 1-12, wherein the carrier protein is an albumin.

**14.** The fusion polypeptide according to claim 13, wherein the carrier protein is a human serum albumin (HSA).

**15.** The fusion polypeptide according to claim 14, wherein the loop is selected from the group consisting of loops at D56-L66, A92-P96, D129-E131, Q170-A172, K281-L283, V293-L305, E311-S312, E321-A322, A362-D365, L398-E400, K439-R445, E465-D471, P537-E542 and A561-T566, the positions are numbered by reference to SEQ ID NO: 16.

**16.** The fusion polypeptide according to claim 15, wherein the polypeptide of interest is a circularly permutated IL-2, the loop is selected from the group consisting of loops at D56-L66, V293-L305 and A362-D365 of the HSA.

**17.** The fusion polypeptide according to claim 15, wherein the insertion site of the polypeptide of interest is selected from the group consisting of D56, A300, C361 and A362 of the HSA.

**18.** A fusion polypeptide, comprising a carrier protein HSA and a circularly permutated IL-2, wherein the circularly permutated IL-2 comprises four α-helical bundles of, from N-terminal to C-terminal, H3, H4, H1 and H2, or H4, H1, H2 and H3; and wherein the circularly permutated IL-2 is inserted into a loop of the HSA, the loop is selected from the group consisting of loops at D56-L66, A92-P96, D129-E131, Q170-A172, K281-L283, V293-L305, E311-S312, E321-A322, A362-D365, L398-E400, K439-R445, E465-D471, P537-E542 and A561-T566, the positions are numbered by reference to SEQ ID NO: 16, the HSA shields the CD25 binding site of the circularly permutated IL-2, thereby blocking the accessibility of the site.

**19.** The fusion polypeptide according to claim 18, wherein the circularly permutated IL-2 comprises an amino acid sequence of SEQ ID NO: 2, 3, 4 or 5.

**20.** The fusion polypeptide of claim 18 or 19, wherein the loop is selected from the group consisting of loops at D56-L66, V293-L305 and A362-D365 of the HSA.

**21.** The fusion polypeptide according to any one of claims 18-19, wherein the insertion site of the circularly permutated IL-2 is selected from the group consisting of D56, A300, C361 and A362 of the HSA.

**22.** A fusion polypeptide, comprising a carrier protein HSA and a circularly permutated IL-15, wherein the circularly permutated IL-15 comprises four α-helical bundles of, from N-terminal to C-terminal, H3, H4, H1 and H2; and wherein the circularly permutated IL-15 is inserted into a loop of the HSA, the loop is selected from the group consisting of loops at D56-L66, A92-P96, D129-E131, Q170-A172, K281-L283, V293-L305, E311-SS312, E321-A322, A362-D365, L398-E400, K439-R445, E465-D471, P537-E542 and A561-T566, the positions are numbered by reference to SEQ ID NO: 16, the HSA shields the CD215 binding site of the circularly permutated IL-15, thereby blocking the accessibility of the site.

**23.** The fusion polypeptide according to claim 22, wherein the circularly permutated IL-15 comprises an amino acid sequence of SEQ ID NO: 7.

**24.** A fusion polypeptide, comprising an amino acid sequence of one of SEQ ID NOs: 8-11.

**25.** A pharmaceutical composition, comprising the fusion polypeptide according to any one of claims 1-24.

**26.** Use of the fusion polypeptide according to any one of claims 1-24 for the manufacture of a medicament for treating a cancer.

27. Use of the fusion polypeptide according to any one of claims 1-24 for the manufacture of a medicament for activating an immune cell or improving proliferation of an immune cell.

28. The use according to claim 27, wherein the immune cell is T cell or NK cell.

29. An isolated polynucleotide, encoding the fusion polypeptide according to claims 1-24.

30. An expression vector, comprising the polynucleotide according to claim 29.

31. A host cell, comprising the polynucleotide according to claim 29 or the expression vector according to claim 30.

FIG. 1

FIG. 2

FIG. 3

structural model

SDS-PAGE

analytic sieve chromatography

# FIG. 4

FIG. 5

structural model

SDS-PAGE

analytic sieve chromatography

# FIG. 6

FIG. 7

A

Fc hole    Fc knob

CD25
extracellular domain    permutated IL-2

structural model

B

Reduced    Non-reduced

SDS-PAGE

C

analytic sieve chromatography

# FIG. 8

A

Fc knob     Fc hole

CD25
extracellular domain

permutated IL-2

structural model

B

Reduced     Non-reduced

C

M₁     1     2

200 kDa---
116 kDa---
97 kDa---
66 kDa---
44 kDa---
29 kDa---
20 kDa---
14 kDa---
6 kDa---

SDS-PAGE

analytic sieve chromatography

# FIG. 9

cellular activity assay

FIG. 10

cellular activity assay

FIG. 11

cellular activity assay

FIG. 12

cellular activity assay

FIG. 13

cellular activity assay

FIG. 14

cellular activity assay

FIG. 15

FIG. 16

FIG. 17

## INTERNATIONAL SEARCH REPORT

| International application No. |
| --- |
| **PCT/CN2021/105187** |

**A. CLASSIFICATION OF SUBJECT MATTER**

C07K 19/00(2006.01)i; C12N 15/62(2006.01)i; A61K 38/16(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS, DWPI, SIPOABS, CNKI, NCBI, ISI Web of Science: 蛋白, 载体, 白蛋白, 清蛋白, 白细胞介, 白介, 插, 环, 螺旋, 掩, 蔽, protein, carrier, albumin, BSA, HSA, insert+, heli+, loop, interleukin, IL, mask

**C. DOCUMENTS CONSIDERED TO BE RELEVANT**

| Category* | Citation of document, with indication, where appropriate, of the relevant passages | Relevant to claim No. |
| --- | --- | --- |
| Y | CN 110724198 A (SHANGHAI YICHEN MEDICINE TECH. CO., LTD.) 24 January 2020 (2020-01-24) entire document, in particular claims and abstract | 1-31 |
| Y | WO 2019222283 A1 (HARPOON THERAPEUTICS INC.) 21 November 2019 (2019-11-21) entire document, in particular claims and abstract | 1-31 |

☐ Further documents are listed in the continuation of Box C.  ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **22 July 2021** | **12 October 2021** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/105187**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

   ☑ in the form of an Annex C/ST.25 text file.

   ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

   ☐ in the form of an Annex C/ST.25 text file (Rule 13*ter*.1(a)).

   ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

INTERNATIONAL SEARCH REPORT
Information on patent family members

International application No.

**PCT/CN2021/105187**

| Patent document cited in search report | | | Publication date (day/month/year) | Patent family member(s) | | | Publication date (day/month/year) |
|---|---|---|---|---|---|---|---|
| CN | 110724198 | A | 24 January 2020 | WO | 2020015677 | A1 | 23 January 2020 |
| WO | 2019222283 | A1 | 21 November 2019 | CA | 3100327 | A1 | 21 November 2019 |
| | | | | CN | 112513083 | A | 16 March 2021 |
| | | | | AU | 2019271138 | A1 | 07 January 2021 |
| | | | | KR | 20210020903 | A | 24 February 2021 |
| | | | | EP | 3794038 | A1 | 24 March 2021 |
| | | | | SG | 11202011330 P | A | 30 December 2020 |

Form PCT/ISA/210 (patent family annex) (January 2015)

42

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAMBROOK et al.** Molecular Cloning: A Laboratory Manual. Cold Spring Harbor Laboratory, Cold Spring Harbor Laboratory Press, 1989
- **SAMBROOK et al.** Molecular Cloning, A Laboratory Manual. Cold Spring Harbor Press, 1989
- Current Protocols in Molecular Biology. John Wiley & Sons, 1995
- **KENNETH, A et al.** Chemical Stability of Pharmaceuticals: A Handbook for Pharmacists
- **PETERS et al.** *Pharmacokinetc analysis: A Practical Approach,* 1996
- **M GIBALDI ; D PERRON.** Pharmacokinetics. Marcel Dekker, 1982
- **CHARYCH et al.** *Clin Canc Res,* 2016